(19) Europäisches Patentamt
European Patent Office
Office européen des brevets

(11) Veröffentlichungsnummer : **0 420 798 B1**

# (12) EUROPÄISCHE PATENTSCHRIFT

(45) Veröffentlichungstag der Patentschrift : **23.03.94 Patentblatt 94/12**

(51) Int. Cl.[5] : **A61K 47/18,** A61L 2/00

(21) Anmeldenummer : **90810692.5**

(22) Anmeldetag : **12.09.90**

## (54) Antimikrobielle Zusammensetzungen.

(30) Priorität : **21.09.89 CH 3446/89**

(43) Veröffentlichungstag der Anmeldung : **03.04.91 Patentblatt 91/14**

(45) Bekanntmachung des Hinweises auf die Patenterteilung : **23.03.94 Patentblatt 94/12**

(84) Benannte Vertragsstaaten : **AT BE CH DE DK ES FR GB GR IT LI LU NL SE**

(56) Entgegenhaltungen :
**EP-A- 0 242 328**
**EP-A- 0 306 984**
**GB-A- 1 472 084**

(56) Entgegenhaltungen :
**ARZNEIMITTEL FORSCHUNG, Band 9, Nr. 10, Oktober 1959, Seiten 622-625; H. GOETH et al.: "Die antimikrobiellen Eigenschaften von Dodecyl-di(beta-oxyäthyl)-benzyl-ammonium-chlorid"**
**Deutsches Arzneibuch, 9. Ausgabe 1986, S. 17-19**

(73) Patentinhaber : **Ciba Vision AG, Hettlingen
Riethofstrasse 1
CH-8442 Hettlingen (CH)**

(72) Erfinder : **Kis, Gyoergy Lajos, Dr.
Keberlistrasse 21
CH-8273 Triboltingen (CH)**

(74) Vertreter : **Schluep, Hans-Peter et al
c/o CIBA GEIGY AG Patentabteilung Postfach
CH-4002 Basel (CH)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Die Erfindung betrifft antimikrobielle ophthalmische Zusammensetzungen, die bestimmte quaternäre Ammoniumsalze als Konservierungsmittel enthalten.

Die Erfindung betrifft insbesondere antimikrobielle ophthalmische Zusammensetzungen, die mit der menschlichen Tränenflüssigkeit isotonisch sind enthaltend mindestens eine Verbindung der Formel I,

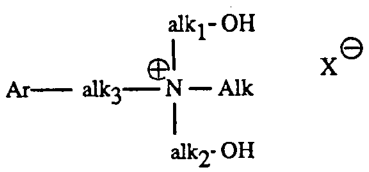

(I)

worin Ar Aryl bedeutet, Alk für Alkyl steht, $alk_1$, $alk_2$ und $alk_3$ unabhängig voneinander Alkylen bedeuten und $X^{\ominus}$ für ein ophthalmisch annehmbares Anion steht, und einen oder mehrere ophthalmisch verträgliche bzw. Kontaktlinsen-verträgliche Hilfsstoffe.

Gegenstand der Erfindung sind zum einen Augenarzneimittel, z.B. Augentropfen, Augengele oder Augensalben, die bestimmte quaternäre Ammoniumsalze als Konservierungsmittel enthalten, sowie die Verwendung von diesen quaternären Ammoniumsalzen zur Konservierung von Augenarzneimitteln.

Zum anderen betrifft die Erfindung z.B. auch Lösungen zur Behandlung von Kontaktlinsen, die bestimmte quaternäre Ammoniumsalze als Konservierungsmittel enthalten, sowie die Verwendung von diesen quaternären Ammoniumsalzen zur Konservierung von Kontaktlinsen.

Arzneimittel zur Behandlung von Erkrankungen im Auge werden häufig in flüssiger Form formuliert und in Tropfenform verabreicht. Bei Augentropfen ist in den meisten europäischen und anderen Ländern eine Konservierung der Präparate vorgeschrieben, d.h. ein Schutz gegen einen Befall und eine anschliessende Vermehrung von Mikroorganismen durch den Zusatz wenigstens einer Komponente, die in der Lage ist, eine sekundäre Kontamination zu verhindern oder sie zumindest einzudämmen. Weiterhin werden Augenarzneimittel häufig als Gel formuliert. Für wässrige Gele gilt das gleiche bezüglich Konservierung.

Es ist bekannt, dass N-Alkyl-N-arylalkyl-N,N-bis-(hydroxyalkyl)-ammoniumsalze als Desinfektionsmittel verwendet werden können. Insbesondere die Substanz Benzoxoniumchlorid ≙ N-Benzyl-N-(n-dodecyl)-N,N-bis-(2-hydroxyethyl)-ammoniumchlorid] wird nunmehr seit über 30 Jahren [s. Arzneimittelforschung 9,622-625(1959)] auf vielen Gebieten zur Desinfektion eingesetzt. Beispielsweise verwendet man Benzoxoniumchlorid in Gurgellösungen zur Desinfektion des Mund- und Rachenraums, bei Laryngotracheitis, gegen dentale Plaque, gegen Karies, zur Hautdesinfektion, insbesondere der Hände in der Chirurgie, für dermatologisch-kosmetische Zwecke oder zur Desinfektion im Krankenhaus, z.B. von medizinischen Instrumenten. Darüber hinaus wird Benzoxoniumchlorid auch als veterinäres Antiseptikum, z.B. als Fungizid und Bakterizid für den Euter oder zur Behandlung von Dermatomykosen bei Pferden eingesetzt. Weitere Einsatzgebiete von Benzoxoniumchlorid sind: Desinfektion in der Nahrungs- und Getränkeindustrie, in der Weinindustrie und in Schlachthöfen, Desinfektion von Tierhäuten, Bakterizid für Textilfasern und Bakterizid und Fungizid für Zuckerrüben.

Es ist ferner bekannt, Augenarzneimittel durch quaternäre Ammoniumsalze zu konservieren (s. z.B. EP-A-306 984). Praktische Anwendung zur Konservierung von Augenarzneimitteln haben - offenbar aufgrund der vielfältigen Anforderungen, die erfüllt sein müssen - nur ganz wenige quaternäre Ammoniumsalze gefunden, und zwar insbesondere die drei in EP-A-306 984 explizit genannten Substanzen Cetyltrimethylammoniumbromid, Cetylpyridiniumchlorid und Benzalkoniumchlorid (vgl. auch EP-A-242 328). Das Konservierungsmittel der Wahl für Augenarzneimittel ist zur Zeit ganz eindeutig das letztgenannte Benzalkoniumchlorid (s. z.B. EP-A-306 984, Seite 2, Zeilen 31-33), welches die folgende Struktur besitzt: N-Benzyl-N-($C_8$-$C_{18}$-alkyl)-N,N-dimethylammoniumchlorid.

Der ganz kleinen Zahl von quaternären Ammoniumsalzen, die in Augenarzneimitteln zur Konservierung verwendet werden, steht eine Vielzahl von bekannten quaternären Ammoniumsalzen ("Quats") gegenüber, die auf anderen Gebieten zur Konservierung eingesetzt werden.

Eine Aufgabe der vorliegenden Erfindung ist es, weitere quaternäre Ammoniumsalze aufzufinden, die zur Konservierung von Augenarzneimitteln geeignet sind. Damit würde die bislang sehr kleine Palette von möglichen Konservierungsmitteln für den Hersteller von Augenarzneimitteln erweitert, und letzterer hätte mehr Alternativen bei der Konservierung seiner Formulierungen. Die genannte Aufgabe wird gelöst durch den Befund, dass die N-Alkyl-N-arylalkyl-N,N-bis-(hydroxyalkyl)-ammoniumsalze der Formel I, zu denen auch Benzoxoniumchlorid gehört, sich hervorragend zur Konservierung von Augenarzneimitteln eignen. Dieser Befund ist keinesfalls naheliegend, denn es sind Hunderte von quaternären Ammoniumsalzen zur Desinfektion allgemein bekannt, von welchen sich überraschenderweise eine ganz kleine Gruppe, nämlich die Verbindungen der Formel I, als zur Konservierung von Augenarzneimitteln geeignet erwiesen hat. Wie oben angegeben wird eine Verbindung der Formel I, Benzoxoniumchlorid, bereits seit über 30 Jahren auf vielen Gebieten zur Desinfektion eingesetzt. Sie ist aber niemals zur Konservierung von Augenarzneimitteln vorgeschlagen worden. Es lag daher nicht nahe, die Verbindungen der Formel I einschliesslich Benzoxoniumchlorid zur Konservierung von Augenarzneimitteln zu verwenden.

Eine weitere Aufgabe der Erfindung ist es, bestimmte quaternäre Ammoniumsalze aufzufinden, die besser als Benzalkoniumchlorid zur Konservierung von Augenarzneimitteln geeignet sind. Wie oben schon erläutert, ist Benzalkoniumchlorid, neben Chlorhexidin, Thiomersal und Chlorobutanol, wohl das derzeit weltweit am häufigsten verwendete Konservierungsmittel für Augenarzneimittel. Es ist aber nun keineswegs so, dass Benzalkoniumchlorid ideal ist und in jeder Hinsicht befriedigt. Insbesondere können bei Langzeitanwendung, die z.B. bei der Behandlung von Glaukom, Katarakt oder "trockenem Auge" erforderlich sein kann, Verträglichkeitsprobleme am Auge auftreten, z.B. Veränderungen des kornealen und konjunktivalen Epithels und bei den Augenlidern. Die Aufgabe, Konservierungsmittel für Augenarzneimittel mit verbesserter Verträglichkeit bei Langzeitanwendung zu finden, wird durch die Bereitstellung von Augenarzneimitteln, die zur Konservierung Verbindungen der Formel I enthalten, gelöst.

Die vorliegende Erfindung betrifft demnach ein Augenarzneimittel, die mit der menschlichen Tränenflüssigkeit isotionisch sind, enthaltend mindestens eine Verbindung der Formel I,

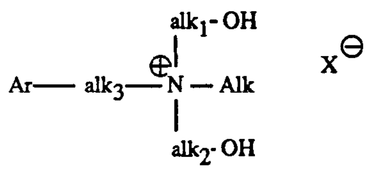

(I)

worin Ar Aryl bedeutet, Alk für Alkyl steht, $alk_1$, $alk_2$ und $alk_3$ unabhängig voneinander Alkylen bedeuten und $X^{\ominus}$ für ein ophthalmisch annehmbares Anion steht, und einen oder mehrere ophthalmisch verträgliche Hilfsstoffe.

Aryl ist Phenyl oder Naphthyl, wie 1- oder 2-Naphthyl. Die Phenyl- und Naphthylreste können unsubstituiert oder substituiert sein, insbesondere wie nachfolgend für Phenyl angegeben. Aryl ist bevorzugt Phenyl, das unsubstituiert oder durch Niederalkyl, Niederalkoxy, Halogen und/oder Trifluormethyl substituiert ist, und in erster Linie Phenyl.

Niederalkyl bedeutet $C_1$-$C_7$-Aryl, z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Niederalkoxy bedeutet $C_1$-$C_7$-Alkoxy, z.B. n-Propoxy, Isopropoxy, n-Butoxy oder tert-Butoxy, vorzugsweise Ethoxy, und vor allem Methoxy.

Halogen steht insbesondere für Fluor, Chlor oder Brom, kann aber auch Iod bedeuten.

Die Aryl- und Alkylengruppen Alk, $alk_1$, $alk_2$ und $alk_3$ sind vorzugsweise geradkettig, können aber auch verzweigt sein.

Die Alkylgruppe Am bedeutet $C_1$-$C_{20}$-Alkyl und steht z.B. für Methyl, Ethyl, n-Propyl, n-Butyl, sek-Butyl, n-Pentyl, n-Hexyl, n-Heptyl, n-Nonadecyl, n-Eicosyl, n-Heneicosyl, n-Docosyl, n-Tricosyl, n-Tetracosyl oder n-Hexacosyl, bevorzugt für n-Octyl, n-Nonyl, n-Decyl, n-Undecyl, n-Tridecyl, n-Tetradecyl, n-Pentadecyl, n-Hexadecyl, n-Heptadecyl oder n-Octadecyl, und in erster Linie für n-Dodecyl.

Die Alkylgruppe Alk steht insbesondere für $C_1$-$C_{18}$-Alkyl und in erster Linie für $C_8$-$C_{18}$-Alkyl.

"Gemischtes $C_8$-$C_{18}$-Alkyl" bedeutet ein Gemisch bestehend im wesentlichen aus n-Octyl, n-Decyl, n-Dodecyl, n-Tetradecyl, n-Hexadecyl und n-Octadecyl, welches 40-60 %, insbesondere ungefähr 50 %, der n-Dodecylkomponente enthält.

Die Alkylengruppen $alk_1$, $alk_2$ und $alk_3$ bedeuten $C_1$-$C_7$-Alkylen und stehen z.B. für Methylen, 1,2-Ethylen, 1,3-Propylen, 1,4-Butylen, 2,4-Butylen, 1,5-Pentylen, 1,6-Hexylen oder 1,7-Heptylen.

Die Alkylengruppen $alk_1$ und $alk_2$ stehen unabhängig voneinander bevorzugt für $C_2$-$C_4$-Alkylen, sind in erster Linie miteinander identisch und stehen vor allen Dingen für 1,2-Ethylen.

Die Alkylengruppe $alk_3$ steht bevorzugt für $C_1$-$C_4$-Alkylen und in erster Linie für Methylen.

Das Anion $X^{\ominus}$ leitet sich von einer ophthalmisch annehmbaren Säure ab. Ophthalmisch annehmbare Säuren sind z.B. starke Mineralsäuren, wie Salzsäure, Bromwasserstoffsäure, Schwefelsäure oder Phosphorsäure, oder organische Säuren, insbesondere aliphatische oder aromatische Carbonsäuren, z.B. Essig-, Propion-, Milch-, Wein-, Zitronen-, Malein-, Fumar-, Hydroxymalein-, Phenylessig-, Glukon- oder Nikotinsäure; oder andere saure organische Substanzen, z.B. Ascorbinsäure. Ferner kommen z.B. auch Aminosäuren, etwa Arginin oder Lysin, in Frage. Bevorzugt bedeutet das Anion $X^{\ominus}$ Bromid und in erster Linie Chlorid.

Die Verbindungen der Formel I zeichnen sich als Konservierungsmittel für Augenarzneimittel unter anderem dadurch aus, dass sie eine ausgezeichnete konservierende Wirkung besitzen und auch bei Langzeitanwendung vom Auge gut vertragen werden.

Die konservierende Wirkung der Verbindungen der Formel I wird z.B. aus den Ergebnissen des folgenden Konservierungsmittelbelastungstests deutlich:

Konservierungsmittelbelastungstest, Substanz: Benzoxoniumchlorid, 0,1 mg/ml, pH 6

| Kontaktzeit mit dem Mikroorganismus | Belastung durch folgende Mikroorganismen: E. coli $1{,}5 \cdot 10^6$/ml | St. aureus $1{,}4 \cdot 10^6$/ml | Ps. aeruginosa $1{,}1 \cdot 10^6$/ml | C. albicans $9{,}9 \cdot 10^5$/ml |
|---|---|---|---|---|
| 10 min | 99,99 % | k. W. | k. W. | 99,97 % |
| 6 h | k. W. | k. W. | k. W. | k. W. |
| 24 h | k. W. | k. W. | k. W. | k. W. |

Angaben in %: Reduktion des Wachstums

k.W.: kein Wachstum

Die überraschenderweise verbesserte Augenverträglichkeit der Verbindungen der Formel I im Vergleich zu Benzalkoniumchlorid wird z.B. aus den Ergebnissen der im folgenden beschriebenen 5-Tage-Verträglichkeitsprüfung beim Tier deutlich: Verglichen wurden jeweils 0,01 %-ige Lösungen von Benzoxoniumchlorid und Benzalkoniumchlorid. Diese Lösungen wurden 5 Tage lang fünfmal (in 90 Minuten-Invervallen) in den Konjunktivalsack von Kaninchen eingetropft. Am Ende des Versuchs wurden die behandelten Augen auf Klinische Befunde, wie z.B. Rötungen der Konjunktiva, Korneatrübungen, Sekretion und Bindehautschwellungen, untersucht. Dabei ergaben sich die folgenden Resultate:

Brenzoxoniumchlorid (≙ Verbindung der Formel I), 0,01 %
5 Instillationen/Tier/Tag im Abstand von jeweils 90 min keine Befunde
Benzalkoniumchlorid (≙ Vergleichsverbindung), 0,01 %
5 Instillationen/Tier/Tag im Abstand von jeweils 90 min 4 Befunde

Die bessere Augenverträglichkeit der Verbindungen der Formel I im Vergleich zu Benzalkoniumchlorid wird z.B. auch aus den Ergebnissen der im folgenden beschriebenen 4 Wochen dauernden okularen, lokalen Verträglichkeitsstudie beim Kaninchen deutlich: Verglichen wurden jeweils 0,01 %-ige Lösungen von N-Benzyl-N-(gemischtes $C_8$-$C_{18}$-Alkyl)-N,N-bis-(2-hydroxyethyl)-ammoniumchlorid und Benzalkoniumchlorid. Als Versuchstiere dienten pigmentierte Himalayakaninchen. Diesen wurden 4 Wochen lang zehnmal täglich (in 45 Minuten-Intervallen) jeweils 50 µl der entsprechenden Lösung in den Konjunktivalsack des rechten Auges verabreicht. Zur Kontrolle wurde in das linke Auge jeweils eine Vergleichslösung, die kein Konservierungsmittel enthielt, eingetropft. Die Tiere wurden am ersten und am fünften Behandlungstag ophthalmologisch untersucht. Nach Beendigung des Versuchs wurden die Versuchstiere zudem histopathologisch untersucht, und es wurden die folgenden Ergebnisse ermittelt: Bei den mit der erfindungsgemässen Lösung behandelten Tieren, die N-Benzyl-N-(gemischtes $C_8$-$C_{18}$-Alkyl)-N,N-bis-(2-hydroxyethyl)-ammoniumchlorid enthielt, wurden keine okularen Läsionen gefunden. Dagegen wurden bei 4 von 6 mit Benzalkoniumchloridlösung behandelten Tieren Läsionen in der Konjunktiva und eine Akanthose und Hyperkeratose am Rand des unteren rechten Augenlids beobachtet. Die Benzalkoniumchloridlösung verursachte demnach Aenderungen am konjunktivalen Epithel, welche bei der erfindungsgemässen Lösung nicht auftraten. Damit ist überzeugend nachgewiesen, dass die Verbindungen der Formel I eine im Vergleich zu Benzalkoniumchlorid überraschend verbesserte Langzeitverträglichkeit im Auge besitzen.

Die Verbindungen der Formel I werden dem Augenarzneimittel normalerweise in einer Konzentration von 0,0001 % - 0,10 % [jeweils Gewicht Volumen (G/V)], insbesondere 0,001 % - 0,02 % und vor allem 0,002 % - 0,015 %, zugesetzt.

Bevorzugt betrifft die Erfindung ein Augenarzneimittel enthaltend mindestens eine Verbindung der Formel I, worin Ar Phenyl, das unsubstituiert oder durch Niederalkyl, Niederalkoxy, Halogen oder Trifluormethyl substituiert ist, bedeutet, Alk für $C_1$-$C_{20}$-Alkyl steht, $alk_1$ und $alk_2$ unabhängig voneinander $C_2$-$C_4$-Alkylen bedeuten,

$alk_3$ für $C_1$-$C_4$-Alkylen steht, und $X^{\ominus}$ ein ophthalmisch annehmbares Anion bedeutet.

Besonders bevorzugt betrifft die Erfindung ein Augenarzneimittel enthaltend mindestens eine Verbindung der Formel I, worin Ar Phenyl bedeutet, Alk für $C_1$-$C_{18}$-Alkyl steht, $alk_1$ und $alk_2$ 1,2-Ethylen bedeuten, $alk_3$ für Methylen steht, und $X^{\ominus}$ Bromid oder Chlorid bedeutet.

Ganz besonders betrifft die Erfindung ein Augenarzneimittel enthaltend mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht und $X^{\ominus}$ Chlorid bedeutet.

In erster Linie betrifft die Erfindung ein Augenarzneimittel enthaltend N-Benzyl-N-(n-dodecyl)-N,N-bis-(2-hydroxyethyl)-ammoniumchlorid oder N-Benzyl-N-(gemischtes $C_8$-$C_{18}$-Alkyl)-N,N-bis-(2-hydroxyethyl)-ammoniumchlorid.

Vor allen Dingen betrifft die Erfindung ein Augenarzneimittel enthaltend N-Benzyl-N-(n-dodecyl)-N,N-bis-(2-hydroxyethyl)-ammoniumchlorid (≙ Benzoxoniumchlorid).

Eine besondere Ausführungsform der Erfindung besteht in einem Augenarzneimittel enthaltend mindestens eine Verbindung der Formel I wie oben definiert und EDTA oder ein Salz davon, z.B. Dinatrium-EDTA. Falls EDTA, oder ein Salz davon im Augenarzneimittel der Erfindung enthalten ist, liegt es vorzugsweise in einer Konzentration von 0,01-0,2 % (G/V), insbesondere 0,05-0,1 % (G/V), vor.

Die Verbindungen der Formel I, welche den Augenarzneimitteln zugesetzt werden, müssen eine für ophthalmische Zwecke ausreichende Reinheit aufweisen. Vorzugsweise enthält z.B. das eingesetzte Benzoxoniumchlorid maximal 2,5 Gewichts-% an Neben produkten, wobei jedes einzelne Nebenprodukt maximal nur mit 0,5 Gewichts-% vertreten sein darf.

Augenarzneimittel können neben dem Konservierungsmittel z.B. die folgenden Bestandteile enthalten:

(a) gegebenenfalls einen oder mehrere pharmazeutische Wirkstoffe, z.B. steroidale entzündungshemmende Substanzen, z.B. Fluorometholon; nichtsteroidale entzündungshemmende Substanzen, z.B. Diclofenac oder ophthalmologisch annehmbare Salze davon, insbesondere Diclofenac-Natrium (Diclofenac-Na); Antibiotika, z.B. Chinolone, Chloramphenicol oder Gentamicin; Antiglaukom-Wirkstoffe, z.B. Pilocarpin-hydrochlorid oder Pirbuterol-hydrochlorid; keratolytisch wirksame Substanzen, z.B. Vitamin-A-säure; gefässverengende Substanzen (Vasokonstriktoren), z.B. Naphazolinnitrat, Tetrahydrozolin (≙ Tetryzolin), Phenylephrin oder Xylometazolin; Antiallergika, z.B. Isospagluminsäure oder Spagluminsäure und deren Magnesium- oder Natriumsalze; adstringierende Substanzen, z.B. Zinksulfat; Antikatarakt-Wirkstoffe, z.B. Methosorbinil [≙ (2R,4S)-2-Methyl-6-fluor-spiro[chroman-4,4′-imidazolidin]-2′,5′-dion]; oder auch z.B. Heilpflanzenextrakte, z.B. Euphrasiatinktur.

Als Augenarzneimittel, die keinen pharmazeutischen Wirkstoff enthalten, sind beispielhaft bestimmte Tränenersatzformulierungen ("künstliche Tränen", "artificial tears") zu nennen.

Ferner enthalten Augenarzneimittel ophthalmisch verträgliche Hilfsstoffe, z.B.

(b) gegebenenfalls eine oder mehrere Puffersubstanzen, z.B. Borsäure, Borate, etwa Borax ($Na_2B_4O_7 \cdot 10H_2O$), Phosphate, z.B. der $Na_2HPO_4 \cdot 2H_2O/NaH_2PO_4 \cdot 2H_2O$-Puffer, Trometamol (≙ 2-Amino-2-hydroxymethyl-1,3-propandiol ≙ TRIS), oder organische Säuren, z.B. Ascorbin-, Essig-, Propion- oder Zitronensäure;

(c) gegebenenfalls ein oder mehrere Isotonisierungsmittel, z.B. Natriumchlorid, Sorbitol (≙ d-Sorbit), Mannitol oder Glycerin;

(d) gegebenenfalls einen oder mehrere Lösungsvermittler, z.B. Fettsäureglycerin-Polyglykolester, Fettsäurepolyglykolester, Polyethylenglykole, Glycerinether oder Gemische aus diesen Verbindungen. Ein spezielles Beispiel für einen besonders bevorzugten Lösungsvermittler sind Reaktionsprodukte aus Rizinusöl und Ethylenoxid, beispielsweise das Handelsprodukt Cremophor® EL (≙ Polyoxyl 35 Castor Oil). Reaktionsprodukte aus Rizinusöl und Ethylenoxid haben sich als besonders gute Lösungsvermittler mit einer ausgezeichneten Augenverträglichkeit erwiesen.

Weitere Beispiele für Lösungsvermittler sind Luviquat® FC 905 (≙ Copolymerisat aus Vinylimidazolium-methochlorid und Vinylpyrrolidon 95:5, Fa. BASF), Solutol® HS 15 (≙ Polyethylenglykol 660-12-hydroxystearat, Fa. BASF) und Macrogol 400 (≙ Polyethylenglykol 400, Lutrol® E 400, Fa. BASF).

(e) gegebenenfalls Lösungsmittel, um bestimmte Komponenten der Formulierung, z.B. den Wirkstoff oder ätherische Oele, die als Geruchsstoffe verwendet werden, in Lösung zu bringen. Beispielhaft seien genannt: Ethanol abs., Polypropylenglykol oder Polyethylenglykol 400 (≙ Macrogol 400, siehe auch unter Lösungsvermittlern);

(f) gegebenenfalls Antioxidantien, z.B. $\alpha$-Tocopherolacetat oder BHA (≙ 3-tert-Butyl-4-hydroxy-anisol);

(g) gegebenenfalls Verdickungsmittel, die zur Erhöhung der Viskosität der Formulierung dienen, z.B. Methylhydroxypropylcellulose, z.B. Methocel F4M® (Fa. Dow Chemicals), oder das Natriumsalz der Hyaluronsäure;

h) gegebenenfalls starke Verdickungsmittel, die zur Gelbildung führen, z.B. Polyacrylsäure, Carbopol®

934 P (≙ Carboxyvinylpolymer, Fa. BF Goodrich), Polymer JR 30 M® (≙ polymeres quaternäres Ammoniumsalz erhalten durch Reaktion von Hydroxyethylcellulose mit einemTrimethylammonium-substituierten Epoxid, vgl. US-Patent 3,472,840, Fa. Union Carbide), Alginate oder Polyvinylpyrrolidon;
i) gegebenenfalls Komplexbildner, z.B. zur Steigerung der konservierenden Wirkung der erfindungsgemässen Konservierungsmittel und/oder zur Komplexierung und damit Maskierung von eventuellen Schwermetallverunreinigungen, z.B. EDTA oder Salze davon, wie Dinatrium-EDTA;
j) gegebenenfalls Geruchsstoffe, z.B. Orangenblütenöl, Lavendelöl oder Hamameliswasser;
k) gegebenenfalls Substanzen, die den pH-Wert beeinflussen, z.B. Salzsäure oder Natriumhydroxid;
l) gegebenenfalls Farbstoffe, z.B. Fluorescein-Natrium; und
m) normalerweise Wasser für Injektionszwecke bzw. demineralisiertes Wasser.

Im Normalfall bemüht man sich, Augenarzneimittel isotonisch mit der menschlichen Tränenflüssigkeit (≙ 270 bis 330 mosmol/kg, insbesondere 300 mosmol/kg) einzustellen.

Die Erfindung betrifft ferner die Verwendung von mindestens einer Verbindung der Formel I,

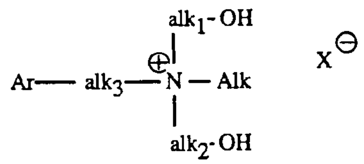

(I)

worin Ar Aryl bedeutet, Alk für Alkyl steht, $alk_1$, $alk_2$ und $alk_3$ unabhängig voneinander Alkylen bedeuten und $X^{\ominus}$ für ein ophthalmisch annehmbares Anion steht, zur Konservierung von Augenarzneimitteln.

Insbesondere betrifft die Erfindung die Verwendung von mindestens einer Verbindung der Formel I, worin Ar Phenyl, das unsubstituiert oder durch Niederalkyl, Niederalkoxy, Halogen oder Trifluormethyl substituiert ist, bedeutet, Alk für $C_1$-$C_{20}$-Alkyl steht, $alk_1$ und $alk_2$ unabhängig voneinander $C_2$-$C_4$-Alkylen bedeuten, $alk_3$ für $C_1$-$C_4$-Alkylen steht, und $X^{\ominus}$ ein ophthalmisch annehmbares Anion bedeutet, zur Konservierung von Augenarzneimitteln.

Besonders bevorzugt betrifft die Erfindung die Verwendung von mindestens einer Verbindung der Formel I, worin Ar Phenyl bedeutet, Alk für $C_1$-$C_{18}$-Alkyl steht, $alk_1$ und $alk_2$ 1,2-Ethylen bedeuten, $alk_3$ für Methylen steht, und $X^{\ominus}$ Bromid oder Chlorid bedeutet, zur Konservierung von Augenarzneimitteln.

Ganz besonders betrifft die Erfindung die Verwendung von mindestens einer Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet, zur Konservierung von Augenarzneimitteln.

In erster Linie betrifft die Erfindung die Verwendung von N-Benzyl-N-(n-dodecyl)-N,N-bis-(2-hydroxyethyl)-ammoniumchlorid oder N-Benzyl-N-(gemischtes $C_8$-$C_{18}$-Alkyl)-N,N-bis-(2-hydroxyethyl)-ammoniumchlorid zur Konservierung von Augenarzneimitteln.

Vor allen Dingen betrifft die Erfindung die Verwendung von N-Benzyl-N-(n-dodecyl)-N,N-bis-(2-hydroxyethyl)-ammoniumchlorid zur Konservierung von Augenarzneimitteln.

Die Erfindung betrifft ferner Lösungen zur Behandlung von Kontaktlinsen, die mit der menschlichen Tränenflüssigkeit isotonisch sind, enthaltend mindestens eine Verbindung der Formel I,

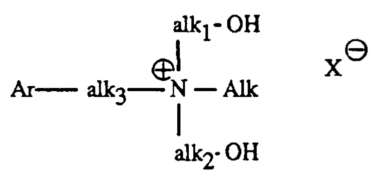

(I)

worin Ar Aryl bedeutet, Alk für Alkyl steht, $alk_1$, $alk_2$ und $alk_3$ unabhängig voneinander Alkylen bedeuten und $X^{\ominus}$ für ein ophthalmisch annehmbares Anion steht, und einen oder mehrere Kontaktlinsen-verträgliche Hilfsstoffe.

Unter Lösungen zur Behandlung von Kontaktlinsen sind insbesondere zu verstehen: (a) Reinigungslösungen für Kontaktlinsen und (b) Conditioner für gasdurchlässige und harte Kontaktlinsen.

Die bevorzugten Untergruppen von Verbindungen der Formel I sind hier die gleichen wie oben bei den Augenarzneimitteln enthaltend Verbindungen der Formel I angegeben.

Insbesonders betrifft daher die Erfindung z.B. Lösungen zur Behandlung von Kontaktlinsen enthaltend mindestens eine Verbindung der Formel I, worin Ar Phenyl bedeutet, Alk für $C_8$-$C_{18}$-Alkyl steht, $alk_1$ und $alk_2$

1,2-Ethylen bedeuten, $alk_3$ für Methylen steht, und $X^{\ominus}$ Chlorid bedeutet.

Eine bevorzugte Reinigungslösung für Kontaktlinsen gemäss der Erfindung enthält: (a) eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet in einer Konzentration (G/V) von 0,005 bis 0,8 %, (b) ein Isotonisierungsmittel, z.B. Natriumchlorid, (c) eine Puffersubstanz, z.B. einen Phosphat-Puffer, (d) einen Komplexbildner, z.B. EDTA oder ein Salz davon, (e) einen Lösungsvermittler, z.B. Aminoxid-Detergentien, z.B. Varox® 365 (Firma Sherex Chemical), oder die Triton®-Serie (≙ Polyethylenglykol-p-isooktylphenylether) der Firma Röhm und Haas, insbesondere Triton® X-100, und gegebenenfalls (f) ein Verdickungsmittel, z.B. schnell lösliche Hydroxyethylcellulose, z.B. Cellosize® QP-40 HEC (Firma Union Carbide).

Ein bevorzugter Conditioner für Kontaktlinsen gemäss der Erfindung enthält: (a) eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet in einer Konzentration (G/V) von 0,002 bis 0,8 %, (b) ein Befeuchtungsmittel, z.B. Polyvinylalkohol, etwa Vinol® 350 PVA (Firma Air Products, Inc.), oder Gemische von Polyvinylalkohol und Polyvinylpyrrolidon (z.B. Vinol® 350 PVA+Plasdone K-90 PVP, Firma GAF), (c) eine Puffersubstanz, z.B. einen Phosphat-Puffer, (d) ein Isotonisierungsmittel, z.B. Natriumchlorid, und gegebenenfalls (e) einen Komplexbildner, z.B. EDTA oder ein Salz davon.

Die oben bei der bevorzugten Reinigungslösung bzw. dem bevorzugten Conditioner für Kontaktlinsen unter (b)-(f) bzw. (b)-(e) angegebenen Komponenten sind Beispiele für Kontaktlinsen-verträgliche Hilfsstoffe.

Die Erfindung betrifft ferner die Verwendung von mindestens einer Verbindung der Formel I,

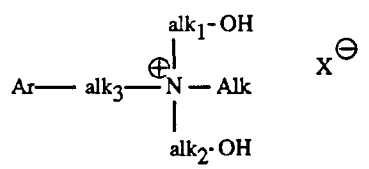

(I)

worin Ar Aryl bedeutet, Alk für Alkyl steht, $alk_1$, $alk_2$ und $alk_3$ unabhängig voneinander Alkylen bedeuten und $X^{\ominus}$ für ein ophthalmisch annehmbares Anion steht, zur Konservierung von Kontaktlinsen.

Die hier bevorzugt verwendeten Untergruppen von Verbindungen der Formel I sind die gleichen wie oben bei den Augenarzneimitteln enthaltend Verbindungen der Formel I angegeben.

Insbesondere betrifft daher die Erfindung z.B. die Verwendung von mindestens einer Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet, zur Konservierung von Kontaktlinsen.

Die antimikrobiellen ophthalmischen Zusammensetzungen gemäss der Erfindung enthalten bevorzugt quaternäre Ammoniumsalze, die aus bestimmten Untergruppen von Verbindungen der Formel I ausgewählt sind. Diese bevorzugten Untergruppen von Verbindungen der Formel I sind die gleichen wie oben bei den Augenarzneimitteln enthaltend Verbindungen der Formel I angegeben.

Insbesondere betrifft daher die Erfindung z.B. auch antimikrobielle ophthalmische Zusammensetzungen enthaltend mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet.

Man verfährt bei der Herstellung von Augenarzneimitteln - wie auch Lösungen zur Behandlung von Kontaktlinsen - gemäss der Erfindung so, dass man eine wirksame, d.h. zur Konservierung ausreichende, Menge von einer oder mehreren Verbindungen der Formel I und die übrigen Bestandteile gemäss konventionellen Methoden vermischt.

Die folgenden Formulierungsbeispiele erläutern die Erfindung:

Beispiel 1: Augentropfen enthaltend Diclofenac-Na

| Bestandteil | | Menge |
|---|---|---|
| Diclofenac-Na | | 1,00 mg |
| Cremophor® EL | | 10,00 mg |
| Luviquat® FC 905 | | 5,00 mg |
| Trometamol | | 6,00 mg |
| Borsäure | | 19,50 mg |
| Dinatrium-EDTA | | 1,00 mg |
| Benzoxoniumchlorid | | 0,10 mg |
| Wasser für Injektionszwecke | ad | 1,00 ml |

Beispiel 2: Augentropfen enthaltend Naphazolinnitrat und Zinksulfat

| Bestandteil | | Menge |
|---|---|---|
| Naphazolinnitrat | | 0,050 mg |
| Zinksulfat | | 0,200 mg |
| Natriumchlorid | | 4,050 mg |
| Benzoxoniumchlorid | | 0,100 mg |
| Methylhydroxypropylcellulose | | 3,000 mg |
| Hamameliswasser | | 40,000 mg |
| Orangenblütenöl | | 5,00 µg |
| Lavendelöl | | 1,80 µg |
| Euphrasiatinktur | | 0,800 mg |
| Ethanol abs. | | 1,393 mg |
| Salzsäure 1N | | 0,100 mg |
| Wasser für Injektionszwecke | ad | 1,000 ml |

Beispiel 3: Augentropfen enthaltend Naphazolinnitrat und Zinksulfat

| Bestandteil | Menge |
|---|---|
| Naphazolinnitrat | 0,0525 mg |
| Zinksulfat | 0,200 mg |
| Borsäure | 7,000 mg |
| Trometamol | 5,000 mg |
| Salzsäure 1N | 34,000 mg |
| Benzoxoniumchlorid | 0,100 mg |
| Methylhydroxypropylcellulose | 3,000 mg |
| Hamameliswasser | 40,000 mg |
| Orangenblütenöl | 5,00 µg |
| Lavendelöl | 1,80 µg |
| Euphrasiatinktur | 0,800 µg |
| Ethanol abs. | 1,393 mg |
| Wasser für Injektionszwecke | ad 1,000 ml |

Beispiel 4: Augentropfen enthaltend Chloramphenicol

| Bestandteil | Menge |
|---|---|
| Chloramphenicol | 6,00 mg |
| Dinatrium-EDTA | 0,10 mg |
| Macrogol 400 | 80,00 mg |
| Trometamol | 0,10 mg |
| Benzoxoniumchlorid | 0,10 mg |
| Cremophor® EL | 4,00 mg |
| Wasser für Injektionszwecke | ad 1,00 ml |

Beispiel 5: Augentropfen enthaltend Chloramphenicol

| Bestandteil | Menge |
|---|---|
| Chloramphenicol | 6,00 mg |
| Dinatrium-EDTA | 0,10 mg |
| Macrogol 400 | 80,00 mg |
| Trometamol | 0,10 mg |
| Benzoxoniumchlorid | 0,10 mg |
| α-Tocopherolacetat | 10,00 mg |
| Solutol® HS 15 | 5,00 mg |
| Wasser für Injektionszwecke | ad 1,00 ml |

Beispiel 6: Augentropfen enthaltend Chloramphenicol

| Bestandteil | Menge |
|---|---|
| Chloramphenicol | 6,00 mg |
| Dinatrium-EDTA | 0,10 mg |
| Macrogol 400 | 80,00 mg |
| Benzoxoniumchlorid | 0,10 mg |
| Wasser für Injektionszwecke | ad 1,00 ml |

Beispiel 7: Augentropfen enthaltend Chloramphenicol

| Bestandteil | Menge |
|---|---|
| Chloramphenicol | 6,00 mg |
| Dinatrium-EDTA | 0,10 mg |
| Macrogol 400 | 80,00 mg |
| Trometamol | 0,10 mg |
| Benzoxoniumchlorid | 0,10 mg |
| Wasser für Injektionszwecke | ad 1,00 ml |

Beispiel 8: Augentropfen enthaltend Chloramphenicol

| Bestandteil | Menge |
|---|---|
| Chloramphenicol | 6,00 mg |
| Dinatrium-EDTA | 0,10 mg |
| Macrogol 400 | 80,00 mg |
| α-Tocopherolacetat | 10,00 mg |
| Benzoxoniumchlorid | 0,10 mg |
| Wasser für Injektionszwecke | ad 1,00 ml |

Beispiel 9: Augentropfen enthaltend Chloramphenicol

| Bestandteil | Menge |
|---|---|
| Chloramphenicol | 6,00 mg |
| Dinatrium-EDTA | 0,10 mg |
| Macrogol 400 | 80,00 mg |
| Cremophor® EL | 4,00 mg |
| Benzoxoniumchlorid | 0,10 mg |
| Wasser für Injektionszwecke | ad 1,00 ml |

Beispiel 10: Augentropfen enthaltend Chloramphenicol

| Bestandteil | | Menge |
|---|---|---|
| Chloramphenicol | | 6,00 mg |
| Dinatrium-EDTA | | 0,10 mg |
| Macrogol 400 | | 80,00 mg |
| Trometamol | | 0,10 mg |
| Benzoxoniumchlorid | | 0,10 mg |
| Solutol® HS 15 | | 5,00 mg |
| Wasser für Injektionszwecke | ad | 1,00 ml |

Beispiel 11: Augengel enthaltend Vitamin-A-säure, Tränenersatz

| Bestandteil | | Menge |
|---|---|---|
| Polymer JR 30 M® | | 12,80 mg |
| Vitamin-A-säure | | 0,15 mg |
| Dinatrium-EDTA | | 1,00 mg |
| Luviquat® FC 905 | | 5,00 mg |
| Benzoxoniumchlorid | | 0,10 mg |
| α-Tocopherolacetat | | 10,00 mg |
| Trometamol | | 0,50 mg |
| Polypropylenglykol | | 60,00 mg |
| Cremophor® EL | | 30,00 mg |
| BHA | | 0,15 mg |
| Wasser für Injektionszwecke | ad | 1,00 ml |

Beispiel 12: Augengel enthaltend Vitamin-A-säure, Tränenersatz

| Bestandteil | | Menge |
|---|---|---|
| Polymer JR 30 M® | | 12,80 mg |
| Vitamin-A-säure | | 0,15 mg |
| Dinatrium-EDTA | | 1,00 mg |
| Luviquat® FC 905 | | 5,00 mg |
| Benzoxoniumchlorid | | 0,20 mg |
| α-Tocopherolacetat | | 10,00 mg |
| Trometamol | | 0,50 mg |
| Polypropylenglykol | | 60,00 mg |
| Cremophor® EL | | 30,00 mg |
| BHA | | 0,15 mg |
| Wasser für Injektionszwecke | ad | 1,00 ml |

Beispiel 13: Augengel enthaltend Vitamin-A-säure, Tränenersatz

| Bestandteil | Menge |
|---|---|
| Polymer JR 30 M® | 12,00 mg |
| Vitamin-A-säure | 0,15 mg |
| Dinatrium-EDTA | 1,00 mg |
| Benzoxoniumchlorid | 0,10 mg |
| Trometamol | 0,40 mg |
| Natriumchlorid | 6,00 mg |
| Solutol® HS 15 | 20,00 mg |
| BHA | 0,15 mg |
| Wasser für Injektionszwecke | ad 1,00 ml |

Beispiel 14: Augengel enthaltend Vitamin-A-säure, Tränenersatz

| Bestandteil | Menge |
|---|---|
| Polymer JR 30 M® | 12,00 mg |
| Vitamin-A-säure | 0,15 mg |
| Dinatrium-EDTA | 1,00 mg |
| Benzoxoniumchlorid | 0,10 mg |
| Trometamol | 0,40 mg |
| Natriumchlorid | 6,00 mg |
| Solutol® HS 15 | 20,00 mg |
| Luviquat® FC 905 | 5,00 mg |
| α-Tocopherolacetat | 10,00 mg |
| Wasser für Injektionszwecke | ad 1,00 ml |

Beispiel 15: Augengel enthaltend Vitamin-A-säure, Tränenersatz

| Bestandteil | Menge |
|---|---|
| Polymer JR 30 M® | 12,00 mg |
| Vitamin-A-säure | 0,15 mg |
| Dinatrium-EDTA | 1,00 mg |
| Luviquat® FC 905 | 5,00 mg |
| Benzoxoniumchlorid | 0,10 mg |
| Trometamol | 0,40 mg |
| Natriumchlorid | 6,00 mg |
| Solutol® HS 15 | 20,00 mg |
| Wasser für Injektionszwecke | ad 1,00 ml |

Beispiel 16: Augengel enthaltend Vitamin-A-säure, Tränenersatz

| Bestandteil | Menge |
|---|---|
| Polymer JR 30 M® | 12,00 mg |
| Vitamin-A-säure | 0,15 mg |
| Dinatrium-EDTA | 1,00 mg |
| Benzoxoniumchlorid | 0,10 mg |
| Trometamol | 0,40 mg |
| Natriumchlorid | 6,00 mg |
| Solutol® HS 15 | 20,00 mg |
| Wasser für Injektionszwecke | ad 1,00 ml |

Beispiel 17: Augengel, Tränenersatz

| Bestandteil | Menge |
|---|---|
| Polymer JR 30 M® | 12,00 mg |
| Sorbitol | 43,00 mg |
| Dinatrium-EDTA | 1,00 mg |
| Luviquat® FC 905 | 5,00 mg |
| Benzoxoniumchlorid | 0,10 mg |
| Wasser für Injektionszwecke | ad 1,00 ml |

Beispiel 18: Augengel, Tränenersatz

| Bestandteil | Menge |
|---|---|
| Polyacrylsäure | 4,00 mg |
| Sorbitol | 43,00 mg |
| Dinatrium-EDTA | 1,00 mg |
| Natriumhydroxid (10 % wässrige Lösung) | 1,50 mg |
| Benzoxoniumchlorid | 0,10 mg |
| Wasser für Injektionszwecke | ad 1,00 ml |

Beispiel 19: Augengel enthaltend Vitamin-A-säure, Tränenersatz

| Bestandteil | Menge |
|---|---|
| Polyacrylsäure | 5,00 mg |
| Vitamin-A-säure | 0,15 mg |
| Dinatrium-EDTA | 1,00 mg |
| Benzoxoniumchlorid | 0,10 mg |
| Natriumchlorid | 6,00 mg |
| Solutol® HS 15 | 20,00 mg |
| Wasser für Injektionszwecke | ad 1,00 ml |

Beispiel 20: Augentropfen enthaltend Naphazolinnitrat und Zinksulfat

| Bestandteil | Menge |
|---|---|
| Naphazolinnitrat | 0,0525 mg |
| Zinksulfat | 0,200 mg |
| Borsäure | 7,000 mg |
| Trometamol | 5,000 mg |
| Salzsäure 1N | 34,000 mg |
| N-Benzyl-N-(gemischtes $C_8$-$C_{18}$-Alkyl)-N,N-bis-(2-hydroxyethyl)-ammoniumchlorid | 0,100 mg |
| Methylhydroxypropylcellulose | 3,000 mg |
| Hamameliswasser | 40,000 mg |
| Orangenblütenöl | 5,00 µg |
| Lavendelöl | 1,80 µg |
| Euphrasiatinktur | 0,800 µg |
| Ethanol abs. | 1,393 mg |
| Wasser für Injektionszwecke | ad 1,000 ml |

Beispiel 21: Augentropfen enthaltend Chloramphenicol

| Bestandteil | Menge |
|---|---|
| Chloramphenicol | 6,00 mg |
| Dinatrium-EDTA | 0,10 mg |
| Macrogol 400 | 80,00 mg |
| Trometamol | 0,10 mg |
| N-Benzyl-N-(gemischtes $C_8$-$C_{18}$-Alkyl)-N,N-bis-(2-hydroxyethyl)-ammonium-chlorid | 0,10 mg |
| Cremophor® EL | 4,00 mg |
| Wasser für Injektionszwecke | ad 1,00 ml |

Beispiel 22: Augentropfen enthaltend Chloramphenicol

| Bestandteil | Menge |
|---|---|
| Chloramphenicol | 6,00 mg |
| Dinatrium-EDTA | 0,10 mg |
| Macrogol 400 | 80,00 mg |
| Trometamol | 0,10 mg |
| N-Benzyl-N-(gemischtes $C_8$-$C_{18}$-Alkyl)-N,N-bis-(2-hydroxyethyl)-ammonium-chlorid | 0,10 mg |
| Wasser für Injektionszwecke | ad 1,00 ml |

Beispiel 23: Augentropfen enthaltend Chloramphenicol

| Bestandteil | Menge |
|---|---|
| Chloramphenicol | 6,00 mg |
| Dinatrium-EDTA | 0,10 mg |
| Macrogol 400 | 80,00 mg |
| N-Benzyl-N-(gemischtes $C_8$-$C_{18}$-Alkyl)-N,N-bis-(2-hydroxyethyl)-ammonium-chlorid | 0,10 mg |
| Solutol® HS 15 | 5,00 mg |
| Wasser für Injektionszwecke | ad 1,00 ml |

Beispiel 24: Augentropfen enthaltend Pirbuterol-hydrochlorid

| Bestandteil | Menge |
| --- | --- |
| Pirbuterol-hydrochlorid | 3,00 mg |
| Dinatrium-EDTA | 0,10 mg |
| Benzoxoniumchlorid | 0,10 mg |
| Natriumchlorid | 8,25 mg |
| Natriumhydroxid (1N wässrige Lösung) | 0,10 mg |
| Wasser für Injektionszwecke | ad 1,00 ml |

Beispiel 25: Augentropfen enthaltend Pirbuterol-hydrochlorid

| Bestandteil | Menge |
| --- | --- |
| Pirbuterol-hydrochlorid | 5,00 mg |
| Dinatrium-EDTA | 0,10 mg |
| Benzoxoniumchlorid | 0,10 mg |
| Natriumchlorid | 7,50 mg |
| Natriumhydroxid (1N wässrige Lösung) | 0,10 mg |
| Wasser für Injektionszwecke | ad 1,00 ml |

Beispiel 26: Augentropfen enthaltend Pirbuterol-hydrochlorid

| Bestandteil | Menge |
| --- | --- |
| Pirbuterol-hydrochlorid | 8,00 mg |
| Dinatrium-EDTA | 0,10 mg |
| Benzoxoniumchlorid | 0,10 mg |
| Natriumchlorid | 6,75 mg |
| Natriumhydroxid (1N wässrige Lösung) | 0,10 mg |
| Wasser für Injektionszwecke | ad 1,00 ml |

Beispiel 27: Augengel enthaltend Diclofenac-Na und Gentamicin

| Bestandteil | Menge |
| --- | --- |
| Diclofenac-Na | 1,00 mg |
| Gentamicin | 3,00 mg |
| Cremophor® EL | 10,00 mg |
| Dinatrium-EDTA | 1,00 mg |
| Benzoxoniumchlorid | 0,10 mg |
| Borax ($Na_2B_4O_7 \cdot 10\ H_2O$) | 1,50 mg |
| Borsäure | 13,00 mg |

| | |
|---|---|
| Polymer JR-30® | 12,50 mg |
| Wasser für Injektionszwecke | ad 1,00 ml |

Beispiel 28: Augentropfen enthaltend Diclofenac-Na und Gentamicin

| Bestandteil | Menge |
|---|---|
| Diclofenac-Na | 1,00 mg |
| Gentamicin | 3,30 mg |
| Cremophor® EL | 50,00 mg |
| Borsäure | 13,00 mg |
| Trometamol | 6,00 mg |
| Dinatrium-EDTA | 1,00 mg |
| Benzoxoniumchlorid | 0,10 mg |
| Natriumchlorid | 2,80 mg |
| Wasser für Injektionszwecke | ad 1,00 ml |

Beispiel 29: Reinigungslösung für Kontaktlinsen

| Bestandteil | Menge |
|---|---|
| Benzoxoniumchlorid | 0,01 g |
| Natriumchlorid | 0,36 g |
| $Na_2HPO_4$ | 0,73 g |
| $NaH_2PO_4 \cdot H_2O$ | 0,21 g |
| Dinatrium-EDTA | 0,10 g |
| Varox® 365 | 1,00 ml |
| Triton® X-100 | 0,33 ml |
| Cellosize® QP-40 HEC | 1,50 g |
| Demineralisiertes Wasser | ad 100,00 ml |

Beispiel 30: Konditionierungslösung für gasdurchlässige und harte Kontaktlinsen

| Bestandteil | Menge |
|---|---|
| Benzoxoniumchlorid | 0,005 g |
| Vinol® 350 PVA | 0,500 g |
| Plasdone K-90 PVP | 1,000 g |
| $Na_2HPO_4$ | 0,720 g |
| $NaH_2PO_4 \cdot H_2O$ | 0,220 g |

| | | |
|---|---|---|
| Natriumchlorid | | 0,440 g |
| Dinatrium-EDTA | | 0,100 g |
| Demineralisiertes Wasser | ad | 100,00 ml |

## Patentansprüche

### Patentansprüche für folgende Vertragsstaaten : AT, BE, CH, DE, DK, FR, GB, IT, LI, LU, NL, SE

1. Antimikrobielle ophthalmische Zusammensetzung, die mit der menschlichen Tränenflüssigkeit isotonisch ist, enthaltend mindestens eine Verbindung der Formel I,

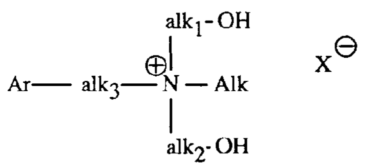

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^\ominus$ für ein ophthalmisch annehmbares Anion steht, und einen oder mehrere ophthalmisch verträgliche bzw. Kontaktlinsen-verträgliche Hilfsstoffe.

2. Antimikrobielle ophthalmische Zusammensetzung gemäss Anspruch 1 enthaltend mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^\ominus$ Chlorid bedeutet.

3. Augenarzneimittel gemäss Anspruch 1 enthaltend mindestens eine Verbindung der Formel I,

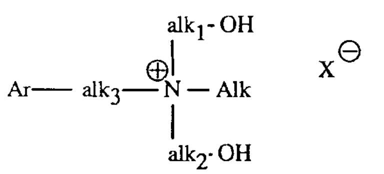

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^\ominus$ für ein ophthalmisch annehmbares Anion steht, und einen oder mehrere ophthalmisch verträgliche Hilfsstoffe.

4. Augenarzneimittel gemäss Anspruch 3 enthaltend mindestens eine Verbindung der Formel I, worin Ar Phenyl, das unsubstituiert oder durch $C_1$-$C_7$-Alkyl, $C_1$-$C_7$-Alkoxy, Halogen oder Trifluormethyl substituiert ist, bedeutet, Alk für $C_1$-$C_{20}$-Alkyl steht, $alk_1$ und $alk_2$ unabhängig voneinander $C_2$-$C_4$-Alkylen bedeuten, $alk_3$ für $C_1$-$C_4$-Alkylen steht, und $X^\ominus$ ein ophthalmisch annehmbares Anion bedeutet.

5. Augenarzneimittel gemäss Anspruch 3 enthaltend mindestens eine Verbindung der Formel I, worin Ar Phenyl bedeutet, Alk für $C_1$-$C_{18}$-Alkyl steht, $alk_1$ und $alk_2$ 1,2-Ethylen bedeuten, $alk_3$ für Methylen steht, und $X^\ominus$ Bromid oder Chlorid bedeutet.

6. Augenarzneimittel gemäss Anspruch 3 enthaltend mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet.

7. Augenarzneimittel gemäss Anspruch 3 enthaltend N-Benzyl-N-(n-dodecyl)-N,N-bis-(2-hydroxyethyl)-ammoniumchlorid oder N-Benzyl-N-(gemischtes $C_8$-$C_{18}$-Alkyl)-N,N-bis-(2-hydroxyethyl)-ammoniumchlorid.

8. Augenarzneimittel gemäss Anspruch 3 enthaltend N-Benzyl-N-(n-dodecyl)-N,N-bis-(2-hydroxyethyl)-ammoniumchlorid.

9. Augenarzneimittel gemäss einem der Ansprüche 3 bis 8, dadurch gekennzeichnet, dass es die Verbindungen der Formel I in einer Konzentration (G/V) von 0,0001 % bis 0,10 % enthält.

10. Augenarzneimittel gemäss einem der Ansprüche 3 bis 8, dadurch gekennzeichnet, dass es die Verbindungen der Formel I in einer Konzentration (G/V) von 0,001 % bis 0,02 % enthält.

11. Augenarzneimittel gemäss einem der Ansprüche 3 bis 8, dadurch gekennzeichnet, dass es die Verbindungen der Formel I in einer Konzentration (G/V) von 0,002 % bis 0,015 % enthält.

12. Augenarzneimittel gemäss einem der Ansprüche 3-11 enthaltend den pharmazeutischen Wirkstoff Diclofenac oder ein ophthalmisch annehmbares Salz davon.

13. Augenarzneimittel gemäss einem der Ansprüche 3-11 enthaltend den pharmazeutischen Wirkstoff Diclofenac-Natrium.

14. Augenarzneimittel gemäss einem der Ansprüche 3-11 enthaltend die pharmazeutischen Wirkstoffe Diclofenac-Natrium und Gentamicin.

15. Augenarzneimittel gemäss einem der Ansprüche 3-11 enthaltend den pharmazeutischen Wirkstoff Naphazolinnitrat.

16. Verwendung von mindestens einer Verbindung der Formel I,

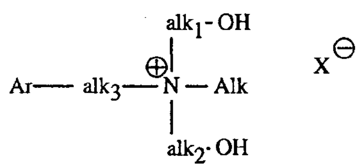

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, zur Konservierung von Augenarzneimitteln.

17. Verwendung gemäss Anspruch 16, dadurch gekennzeichnet, dass mindestens eine Verbindung der Formel I, worin Ar Phenyl, das unsubstituiert oder durch $C_1$-$C_7$-Alkyl, $C_1$-$C_7$-Alkoxy, Halogen oder Trifluormethyl substituiert ist, bedeutet, Alk für $C_1$-$C_{20}$-Alkyl steht, $alk_1$ und $alk_2$ unabhängig voneinander $C_2$-$C_4$-Alkylen bedeuten, $alk_3$ für $C_1$-$C_4$-Alkylen steht, und $X^{\ominus}$ ein ophthalmisch annehmbares Anion bedeutet, angewandt wird.

18. Verwendung gemäss Anspruch 16, dadurch gekennzeichnet, dass mindestens eine Verbindung der Formel I, worin Ar Phenyl bedeutet, Alk für $C_1$-$C_{18}$-Alkyl steht, $alk_1$ und $alk_2$ 1,2-Ethylen bedeuten, $alk_3$ für Methylen steht, und $X^{\ominus}$ Bromid oder Chlorid bedeutet, angewandt wird.

19. Verwendung gemäss Anspruch 16, dadurch gekennzeichnet, dass mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet, angewandt wird.

**20.** Verwendung gemäss Anspruch 16, dadurch gekennzeichnet, dass N-Benzyl-N-(n-dodecyl)-N,N-bis-(2-hydroxyethyl)-ammoniumchlorid oder N-Benzyl-N-(gemischtes $C_8$-$C_{18}$-Alkyl)-N,N-bis-(2-hydroxyethyl)-ammoniumchlorid angewandt wird.

**21.** Verwendung gemäss Anspruch 16, dadurch gekennzeichnet, dass N-Benzyl-N-(n-dodecyl)-N,N-bis-(2-hydroxyethyl)-ammoniumchlorid angewandt wird.

**22.** Verwendung gemäss einem der Ansprüche 16-21, dadurch gekennzeichnet, dass die Verbindungen der Formel I im Augenarzneimittel in einer Konzentration (G/V) von 0,0001 % bis 0,10 % angewandt werden.

**23.** Verwendung gemäss einem der Ansprüche 16-21, dadurch gekennzeichnet, dass die Verbindungen der Formel I im Augenarzneimittel in einer Konzentration (G/V) von 0,001 % bis 0,02 % angewandt werden.

**24.** Verwendung gemäss einem der Ansprüche 16-21, dadurch gekennzeichnet, dass die Verbindungen der Formel I im Augenarzneimittel in einer Konzentration (G/V) von 0,002 % bis 0,015 % angewandt werden.

**25.** Eine Lösung zur Behandlung von Kontaktlinsen, die mit der menschlichen Tränenflüssigkeit isotonisch ist, enthaltend mindestens eine Verbindung der Formel I,

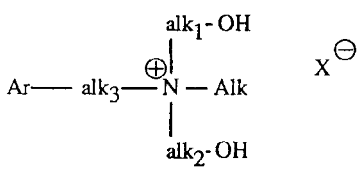

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, und einen oder mehrere Kontaktlinsen-verträgliche Hilfsstoffe.

**26.** Eine Lösung zur Behandlung von Kontaktlinsen gemäss Anspruch 25 enthaltend mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet.

**27.** Eine Reinigungslösung für Kontaktlinsen gemäss Anspruch 26 enthaltend:
(a) eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet in einer Konzentration (G/V) von 0,005 bis 0,8 %,
(b) ein Isotonisierungsmittel,
(c) eine Puffersubstanz,
(d) einen Komplexbildner,
(e) einen Lösungsvermittler, und gegebenenfalls
(f) ein Verdickungsmittel.

**28.** Ein Conditioner für Kontaktlinsen gemäss Anspruch 26 enthaltend
(a) eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet in einer Konzentration (G/V) von 0,002 bis 0,8 %,
(b) ein Befeuchtungsmittel,
(c) eine Puffersubstanz,
(d) ein Isotonisierungsmittel, und gegebenenfalls
(e) einen Komplexbildner.

**29.** Verwendung von mindestens einer Verbindung der Formel I,

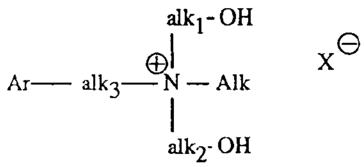

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, zur Konservierung von Kontaktlinsen.

30. Verwendung gemäss Anspruch 29, dadurch gekennzeichnet, dass mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet, angewandt wird.

**Patentansprüche für folgenden Vertragsstaat : GR**

1. Verfahren zur Herstellung einer antimikrobiellen ophthalmischen Zusammensetzung, die mit der menschlichen Tränenflüssigkeit isotonisch ist, enthaltend mindestens eine Verbindung der Formel I,

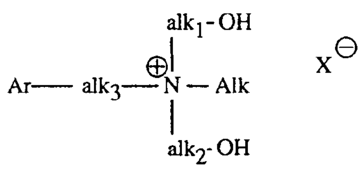

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, und einen oder mehrere ophthalmisch verträgliche bzw. Kontaktlinsen-verträgliche Hilfsstoffe, dadurch gekennzeichnet, dass die Bestandteile der Zusammensetzung miteinander vermischt werden.

2. Verfahren zur Herstellung einer antimikrobiellen ophthalmischen Zusammensetzung gemäss Anspruch 1 enthaltend mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet.

3. Verfahren zur Herstellung eines Augenarzneimittels gemäss Anspruch 1 enthaltend mindestens eine Verbindung der Formel I,

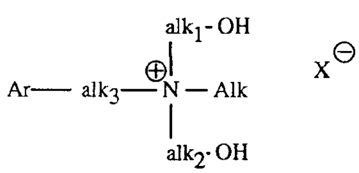

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, und einen oder mehrere ophthalmisch verträgliche Hilfsstoffe, dadurch gekennzeichnet, dass die Bestandteile des Augenarzneimittels miteinander vermischt werden.

4. Verfahren zur Herstellung eines Augenarzneimittels gemäss Anspruch 3 enthaltend mindestens eine Verbindung der Formel I, worin Ar Phenyl, das unsubstituiert oder durch $C_1$-$C_7$-Alkyl, $C_1$-$C_7$-Alkoxy, Halogen oder Trifluormethyl substituiert ist, bedeutet, Alk für $C_1$-$C_{20}$-Alkyl steht, $alk_1$ und $alk_2$ unabhängig voneinander $C_2$-$C_4$-Alkylen bedeuten, $alk_3$ für $C_1$-$C_4$-Alkylen steht, und $X^{\ominus}$ ein ophthalmisch annehmbares Anion bedeutet.

**5.** Verfahren zur Herstellung eines Augenarzneimittels gemäss Anspruch 3 enthaltend mindestens eine Verbindung der Formel I, worin Ar Phenyl bedeutet, Alk für $C_1$-$C_{18}$-Alkyl steht, $alk_1$ und $alk_2$ 1,2-Ethylen bedeuten, $alk_3$ für Methylen steht, und $X^{\ominus}$ Bromid oder Chlorid bedeutet.

**6.** Verfahren zur Herstellung eines Augenarzneimittels gemäss Anspruch 3 enthaltend mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet.

**7.** Verfahren zur Herstellung eines Augenarzneimittels gemäss Anspruch 3 enthaltend N-Benzyl-N-(n-dodecyl)-N,N-bis-(2-hydroxyethyl)-ammoniumchlorid oder N-Benzyl-N-(gemischtes $C_8$-$C_{18}$-Alkyl)-N,N-bis-(2-hydroxyethyl)-ammoniumchlorid.

**8.** Verfahren zur Herstellung eines Augenarzneimittels gemäss Anspruch 3 enthaltend N-Benzyl-N-(n-dodecyl)-N,N-bis-(2-hydroxyethyl)-ammoniumchlorid.

**9.** Verfahren zur Herstellung eines Augenarzneimittels gemäss einem der Ansprüche 3 bis 8, dadurch gekennzeichnet, dass es die Verbindungen der Formel I in einer Konzentration (G/V) von 0,0001 % bis 0,10 % enthält.

**10.** Verfahren zur Herstellung eines Augenarzneimittels gemäss einem der Ansprüche 3 bis 8, dadurch gekennzeichnet, dass es die Verbindungen der Formel I in einer Konzentration (G/V) von 0,001 % bis 0,02 % enthält.

**11.** Verfahren zur Herstellung eines Augenarzneimittels gemäss einem der Ansprüche 3 bis 8, dadurch gekennzeichnet, dass es die Verbindungen der Formel I in einer Konzentration (G/V) von 0,002 % bis 0,015 % enthält.

**12.** Verfahren zur Herstellung eines Augenarzneimittels gemäss einem der Ansprüche 3-11 enthaltend den pharmazeutischen Wirkstoff Diclofenac oder ein ophthalmisch annehmbares Salz davon.

**13.** Verfahren zur Herstellung eines Augenarzneimittels gemäss einem der Ansprüche 3-11 enthaltend den pharmazeutischen Wirkstoff Diclofenac-Natrium.

**14.** Verfahren zur Herstellung eines Augenarzneimittels gemäss einem der Ansprüche 3-11 enthaltend die pharmazeutischen Wirkstoffe Diclofenac-Natrium und Gentamicin.

**15.** Verfahren zur Herstellung eines Augenarzneimittels gemäss einem der Ansprüche 3-11 enthaltend den pharmazeutischen Wirkstoff Naphazolinnitrat.

**16.** Verwendung von mindestens einer Verbindung der Formel I,

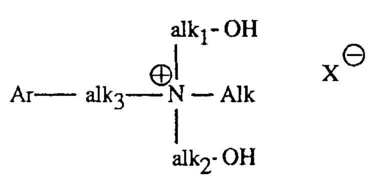

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, zur Konservierung von Augenarzneimitteln.

**17.** Verwendung gemäss Anspruch 16, dadurch gekennzeichnet, dass mindestens eine Verbindung der Formel I, worin Ar Phenyl, das unsubstituiert oder durch $C_1$-$C_7$-Alkyl, $C_1$-$C_7$-Alkoxy, Halogen oder Trifluor-

methyl substituiert ist, bedeutet, Alk für $C_1$-$C_{20}$-Alkyl steht, $alk_1$ und $alk_2$ unabhängig voneinander $C_2$-$C_4$-Alkylen bedeuten, $alk_3$ für $C_1$-$C_4$-Alkylen steht, und $X^{\ominus}$ ein ophthalmisch annehmbares Anion bedeutet, angewandt wird.

**18.** Verwendung gemäss Anspruch 16, dadurch gekennzeichnet, dass mindestens eine Verbindung der Formel I, worin Ar Phenyl bedeutet, Alk für $C_1$-$C_{18}$-Alkyl steht, $alk_1$ und $alk_2$ 1,2-Ethylen bedeuten, $alk_3$ für Methylen steht, und $X^{\ominus}$ Bromid oder Chlorid bedeutet, angewandt wird.

**19.** Verwendung gemäss Anspruch 16, dadurch gekennzeichnet, dass mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet, angewandt wird.

**20.** Verwendung gemäss Anspruch 16, dadurch gekennzeichnet, dass N-Benzyl-N-(n-dodecyl)-N,N-bis-(2-hydroxyethyl)-ammoniumchlorid oder N-Benzyl-N-(gemischtes $C_8$-$C_{18}$-Alkyl)-N,N-bis-(2-hydroxyethyl)-ammoniumchlorid angewandt wird.

**21.** Verwendung gemäss Anspruch 16, dadurch gekennzeichnet, dass N-Benzyl-N-(n-dodecyl)-N,N-bis-(2-hydroxyethyl)-ammoniumchlorid angewandt wird.

**22.** Verwendung gemäss einem der Ansprüche 16-21, dadurch gekennzeichnet, dass die Verbindungen der Formel I im Augenarzneimittel in einer Konzentration (G/V) von 0,0001 % bis 0,10 % angewandt werden.

**23.** Verwendung gemäss einem der Ansprüche 16-21, dadurch gekennzeichnet, dass die Verbindungen der Formel I im Augenarzneimittel in einer Konzentration (G/V) von 0,001 % bis 0,02 % angewandt werden.

**24.** Verwendung gemäss einem der Ansprüche 16-21, dadurch gekennzeichnet, dass die Verbindungen der Formel I im Augenarzneimittel in einer Konzentration (G/V) von 0,002 % bis 0,015 % angewandt werden.

**25.** Eine Lösung zur Behandlung von Kontaktlinsen, die mit der menschlichen Tränenflüssigkeit isotonisch ist, enthaltend mindestens eine Verbindung der Formel I,

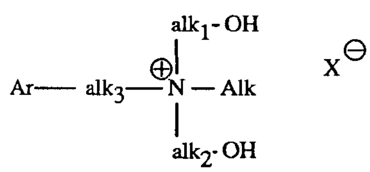

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, und einen oder mehrere Kontaktlinsen-verträgliche Hilfsstoffe.

**26.** Eine Lösung zur Behandlung von Kontaktlinsen gemäss Anspruch 25 enthaltend mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet.

**27.** Eine Reinigungslösung für Kontaktlinsen gemäss Anspruch 26 enthaltend:
(a) eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet in einer Konzentration (G/V) von 0,005 bis 0,8 %,
(b) ein Isotonisierungsmittel,
(c) eine Puffersubstanz,
(d) einen Komplexbildner,
(e) einen Lösungsvermittler, und gegebenenfalls
(f) ein Verdickungsmittel.

**28.** Ein Conditioner für Kontaktlinsen gemäss Anspruch 26 enthaltend

(a) eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet in einer Konzentration (G/V) von 0,002 bis 0,8 %,
(b) ein Befeuchtungsmittel,
(c) eine Puffersubstanz,
(d) ein Isotonisierungsmittel, und gegebenenfalls
(e) einen Komplexbildner.

**29.** Verwendung von mindestens einer Verbindung der Formel I,

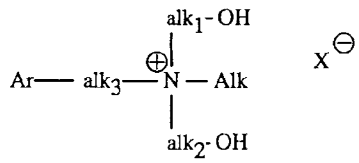

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, zur Konservierung von Kontaktlinsen.

**30.** Verwendung gemäss Anspruch 29, dadurch gekennzeichnet, dass mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet, angewandt wird.

**Patentansprüche für folgenden Vertragsstaat : ES**

**1.** Verfahren zur Herstellung einer antimikrobiellen ophthalmischen Zusammensetzung, die mit der menschlichen Tränenflüssigkeit isotonisch ist, enthaltend mindestens eine Verbindung der Formel I,

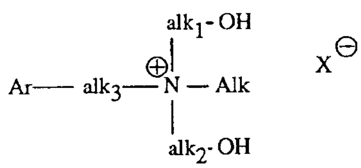

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, und einen oder mehrere ophthalmisch verträgliche bzw. Kontaktlinsen-verträgliche Hilfsstoffe, dadurch gekennzeichnet, dass die Bestandteile der Zusammensetzung miteinander vermischt werden.

**2.** Verfahren zur Herstellung einer antimikrobiellen ophthalmischen Zusammensetzung gemäss Anspruch 1 enthaltend mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet.

**3.** Verfahren zur Herstellung eines Augenarzneimittels gemäss Anspruch 1 enthaltend mindestens eine Verbindung der Formel I,

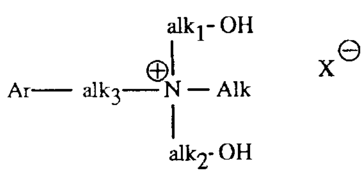

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, und einen oder mehrere ophthalmisch verträgliche Hilfsstoffe, dadurch gekennzeichnet, dass die Bestandteile des Augenarzneimittels miteinander vermischt werden.

**4.** Verfahren zur Herstellung eines Augenarzneimittels gemäss Anspruch 3 enthaltend mindestens eine Verbindung der Formel I, worin Ar Phenyl, das unsubstituiert oder durch $C_1$-$C_7$-Alkyl, $C_1$-$C_7$-Alkoxy, Halogen oder Trifluormethyl substituiert ist, bedeutet, Alk für $C_1$-$C_{20}$-Alkyl steht, $alk_1$ und $alk_2$ unabhängig voneinander $C_2$-$C_4$-Alkylen bedeuten, $alk_3$ für $C_1$-$C_4$-Alkylen steht, und $X^{\ominus}$ ein ophthalmisch annehmbares Anion bedeutet.

**5.** Verfahren zur Herstellung eines Augenarzneimittels gemäss Anspruch 3 enthaltend mindestens eine Verbindung der Formel I, worin Ar Phenyl bedeutet, Alk für $C_1$-$C_{18}$-Alkyl steht, $alk_1$ und $alk_2$ 1,2-Ethylen bedeuten, $alk_3$ für Methylen steht, und $X^{\ominus}$ Bromid oder Chlorid bedeutet.

**6.** Verfahren zur Herstellung eines Augenarzneimittels gemäss Anspruch 3 enthaltend mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet.

**7.** Verfahren zur Herstellung eines Augenarzneimittels gemäss Anspruch 3 enthaltend N-Benzyl-N-(n-dodecyl)-N,N-bis-(2-hydroxyethyl)-ammoniumchlorid oder N-Benzyl-N-(gemischtes $C_8$-$C_{18}$-Alkyl)-N,N-bis-(2-hydroxyethyl)-ammoniumchlorid.

**8.** Verfahren zur Herstellung eines Augenarzneimittels gemäss Anspruch 3 enthaltend N-Benzyl-N-(n-dodecyl)-N,N-bis-(2-hydroxyethyl)-ammoniumchlorid.

**9.** Verfahren zur Herstellung eines Augenarzneimittels gemäss einem der Ansprüche 3 bis 8, dadurch gekennzeichnet, dass es die Verbindungen der Formel I in einer Konzentration (G/V) von 0,0001 % bis 0,10 % enthält.

**10.** Verfahren zur Herstellung eines Augenarzneimittels gemäss einem der Ansprüche 3 bis 8, dadurch gekennzeichnet, dass es die Verbindungen der Formel I in einer Konzentration (G/V) von 0,001 % bis 0,02 % enthält.

**11.** Verfahren zur Herstellung eines Augenarzneimittels gemäss einem der Ansprüche 3 bis 8, dadurch gekennzeichnet, dass es die Verbindungen der Formel I in einer Konzentration (G/V) von 0,002 % bis 0,015 % enthält.

**12.** Verfahren zur Herstellung eines Augenarzneimittels gemäss einem der Ansprüche 3-11 enthaltend den pharmazeutischen Wirkstoff Diclofenac oder ein ophthalmisch annehmbares Salz davon.

**13.** Verfahren zur Herstellung eines Augenarzneimittels gemäss einem der Ansprüche 3-11 enthaltend den pharmazeutischen Wirkstoff Diclofenac-Natrium.

**14.** Verfahren zur Herstellung eines Augenarzneimittels gemäss einem der Ansprüche 3-11 enthaltend die pharmazeutischen Wirkstoffe Diclofenac-Natrium und Gentamicin.

**15.** Verfahren zur Herstellung eines Augenarzneimittels gemäss einem der Ansprüche 3-11 enthaltend den pharmazeutischen Wirkstoff Naphazolinnitrat.

**16.** Verwendung von mindestens einer Verbindung der Formel I,

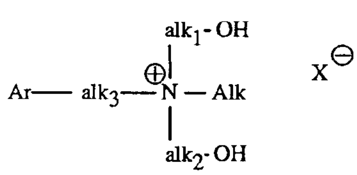

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, zur Konservierung von Augenarzneimitteln.

**17.** Verwendung gemäss Anspruch 16, dadurch gekennzeichnet, dass mindestens eine Verbindung der Formel I, worin Ar Phenyl, das unsubstituiert oder durch $C_1$-$C_7$-Alkyl, $C_1$-$C_7$-Alkoxy, Halogen oder Trifluormethyl substituiert ist, bedeutet, Alk für $C_1$-$C_{20}$-Alkyl steht, $alk_1$ und $alk_2$ unabhängig voneinander $C_2$-$C_4$-Alkylen bedeuten, $alk_3$ für $C_1$-$C_4$-Alkylen steht, und $X^{\ominus}$ ein ophthalmisch annehmbares Anion bedeutet, angewandt wird.

**18.** Verwendung gemäss Anspruch 16, dadurch gekennzeichnet, dass mindestens eine Verbindung der Formel I, worin Ar Phenyl bedeutet, Alk für $C_1$-$C_{18}$-Alkyl steht, $alk_1$ und $alk_2$ 1,2-Ethylen bedeuten, $alk_3$ für Methylen steht, und $X^{\ominus}$ Bromid oder Chlorid bedeutet, angewandt wird.

**19.** Verwendung gemäss Anspruch 16, dadurch gekennzeichnet, dass mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet, angewandt wird.

**20.** Verwendung gemäss Anspruch 16, dadurch gekennzeichnet, dass N-Benzyl-N-(n-dodecyl)-N,N-bis-(2-hydroxyethyl)-ammoniumchlorid oder N-Benzyl-N-(gemischtes $C_8$-$C_{18}$-Alkyl)-N,N-bis-(2-hydroxyethyl)-ammoniumchlorid angewandt wird.

**21.** Verwendung gemäss Anspruch 16, dadurch gekennzeichnet, dass N-Benzyl-N-(n-dodecyl)-N,N-bis-(2-hydroxyethyl)-ammoniumchlorid angewandt wird.

**22.** Verwendung gemäss einem der Ansprüche 16-21, dadurch gekennzeichnet, dass die Verbindungen der Formel I im Augenarzneimittel in einer Konzentration (G/V) von 0,0001 % bis 0,10 % angewandt werden.

**23.** Verwendung gemäss einem der Ansprüche 16-21, dadurch gekennzeichnet, dass die Verbindungen der Formel I im Augenarzneimittel in einer Konzentration (G/V) von 0,001 % bis 0,02 % angewandt werden.

**24.** Verwendung gemäss einem der Ansprüche 16-21, dadurch gekennzeichnet, dass die Verbindungen der Formel I im Augenarzneimittel in einer Konzentration (G/V) von 0,002 % bis 0,015 % angewandt werden.

**25.** Verfahren zur Herstellung einer Lösung zur Behandlung von Kontaktlinsen, die mit der menschlichen Tränenflüssigkeit isotonisch ist, enthaltend mindestens eine Verbindung der Formel I,

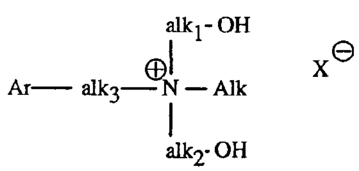

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, und einen oder mehrere Kontaktlinsen-verträgliche Hilfsstoffe, dadurch gekennzeichnet, dass die Bestandteile der Lösung miteinander vermischt werden.

**26.** Verfahren zur Herstellung einer Lösung zur Behandlung von Kontaktlinsen gemäss Anspruch 25 enthaltend mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet.

**27.** Verfahren zur Herstellung einer Reinigungslösung für Kontaktlinsen gemäss Anspruch 26 enthaltend:
(a) eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet in einer Konzentration (G/V) von 0,005 bis 0,8 %,

(b) ein Isotonisierungsmittel,
(c) eine Puffersubstanz,
(d) einen Komplexbildner,
(e) einen Lösungsvermittler, und gegebenenfalls
(f) ein Verdickungsmittel.

28. Verfahren zur Herstellung eines Conditioners für Kontaktlinsen gemäss Anspruch 26 enthaltend
(a) eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet in einer Konzentration (G/V) von 0,002 bis 0,8 %,
(b) ein Befeuchtungsmittel,
(c) eine Puffersubstanz,
(d) ein Isotonisierungsmittel, und gegebenenfalls
(e) einen Komplexbildner.

29. Verwendung von mindestens einer Verbindung der Formel I,

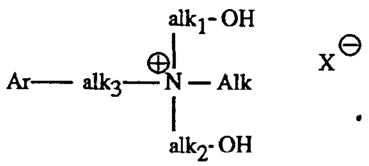

(I)

worin Ar Aryl bedeutet, wobei Aryl für einen der Reste Naphthyl oder Phenyl steht, die unabhängig voneinander unsubstituiert oder durch $C_1$-$C_7$-Alkyl, $C_1$-$C_7$-Alkoxy, Halogen oder Trifluormethyl substituiert sind, Alk für $C_1$-$C_{20}$-Alkyl steht, $alk_1$, $alk_2$ und $alk_3$ unabhängig voneinander $C_1$-$C_7$-Alkylen bedeuten und $X^{\ominus}$ für ein ophthalmisch annehmbares Anion steht, zur Konservierung von Kontaktlinsen.

30. Verwendung gemäss Anspruch 29, dadurch gekennzeichnet, dass mindestens eine Verbindung der Formel I, worin Ar Phenyl bedeutet, Alk für $C_8$-$C_{18}$-Alkyl steht, $alk_1$ und $alk_2$ 1,2-Ethylen bedeuten, $alk_3$ für Methylen steht, und $X^{\ominus}$ Chlorid bedeutet, angewandt wird.

## Claims

### Claims for the following Contracting States : AT, BE, CH, DE, DK, FR, GB, IT, LI, LU, NL, SE

1. An antimicrobial ophthalmic composition which is isotonic with human lachrymal fluid, containing at least one compound of the formula I

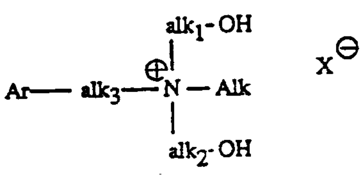

(I)

in which Ar is aryl, with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are $C_1$-$C_7$alkylene, and $X^{\ominus}$ is an ophthalmically acceptable anion, and one or more adjuncts which are opthalmically acceptable and compatible with contact lenses.

2. An antimicrobial ophthalmic composition according to claim 1 containing at least one compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride.

3. An eye medicament according to claim 1 containing at least one compound of the formula I

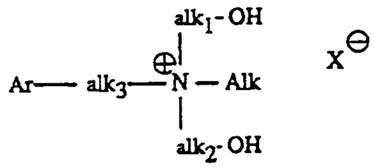

(I)

in which Ar is aryl, with aryl being one of the radicals naphthyl and phenyl which, independently of one another, are unsubstituted or substituted by C1-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are $C_1$-$C_7$alkylene and $X^{\ominus}$ is an ophthalmically acceptable anion, and one or more ophthalmically acceptable adjuncts.

4. An eye medicament according to claim 3 containing at least one compound of the formula I in which Ar is phenyl which is unsubstituted or substituted by $C_1$-$C_7$alkyl, $C_1$-$C_7$alkoxy, halogen or trifluoromethyl, Alk is $C_1$-$C_{20}$alkyl, $alk_1$ and $alk_2$ independently of one another are $C_2$-$C_4$alkylene, $alk_3$ is $C_1$-$C_4$alkylene and $X^{\ominus}$ is an ophthalmically acceptable anion.

5. An eye medicament according to claim 3 containing at least one compound of the formula I in which Ar is phenyl, Alk is $C_1$-$C_{18}$alkyl, $alk_1$ and $alk_2$ are 1,2-ethylene, $alk_3$ is methylene and $X^{\ominus}$ is bromide or chloride.

6. An eye medicament according to claim 3 containing at least one compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride.

7. An eye medicament according to claim 3 containing N-benzyl-N-(n-dodecyl)-N,N-bis(2-hydroxyethyl)ammonium chloride or N-benzyl-N-(mixed $C_8$-$C_{18}$alkyl)-N,N-bis(2-hydroxyethyl)ammonium chloride.

8. An eye medicament according to claim 3 containing N-benzyl-N-(n-dodecyl)-N,N-bis(2-hydroxyethyl)ammonium chloride.

9. An eye medicament according to any one of claims 3 to 8, which contains the compounds of the formula I in a concentration (W/V) of 0.0001 % to 0.10 %.

10. An eye medicament according to any one of claims 3 to 8, which contains the compounds of the formula I in a concentration (W/V) of 0.001 % to 0.02 %.

11. An eye medicament according to any one of claims 3 to 8, which contains the compounds of the formula I in a concentration (W/V) of 0.002 % to 0.015 %.

12. An eye medicament according to any one of claims 3-11 containing the pharmaceutical active ingredient diclofenac or an ophthalmically acceptable salt thereof.

13. An eye medicament according to any one of claims 3-11 containing the pharmaceutical active ingredient diclofenac sodium.

14. An eye medicament according to any one of claims 3-11 containing the pharmaceutical active ingredients diclofenac sodium and gentamicin.

15. An eye medicament according to any one of claims 3-11 containing the pharmaceutical active ingredient naphazoline nitrate.

16. Use of at least one compound of the formula I

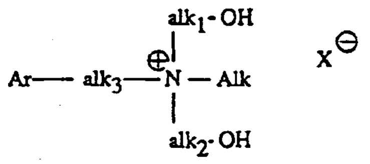

(I)

in which Ar is aryl, with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are $C_1$-$C_7$alkylene and $X^{\ominus}$ is an opthalmically acceptable anion, for the preservation of eye medicaments.

**17.** Use according to claim 16, wherein at least one compound of the formula I in which Ar is phenyl which is unsubstituted or substituted by $C_1$-$C_7$alkyl, $C_1$-$C_7$alkoxy, halogen or trifluoromethyl, Alk is $C_1$-$C_{20}$alkyl, $alk_1$ and $alk_2$ independently of one another are $C_2$-$C_4$alkylene, $alk_3$ is $C_1$-$C_4$alkylene and $X^{\ominus}$ is an ophthalmically acceptable anion, is used.

**18.** Use according to claim 16, wherein at least one compound of the formula I in which Ar is phenyl, Alk is $C_1$-$C_{18}$alkyl, $alk_1$ and $alk_2$ are 1,2-ethylene, $alk_3$ is methylene and $X^{\ominus}$ is bromide or chloride, is used.

**19.** Use according to claim 16, wherein at least one compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride, is used.

**20.** Use according to claim 16, wherein N-benzyl-N-(n-dodecyl)-N,N-bis(2-hydroxyethyl)ammonium chloride or N-benzyl-N-(mixed $C_8$-$C_{18}$alkyl)-N,N-bis(2-hydroxyethyl)ammonium chloride is used.

**21.** Use according to claim 16, wherein N-benzyl-N-(n-dodecyl)-N,N-bis(2-hydroxyethyl)ammonium chloride is used.

**22.** Use according to any one of claims 16-21, wherein the compounds of the formula I are used in the eye medicament in a concentration (W/V) of 0.0001 % to 0.10 %.

**23.** Use according to any one of claims 16-21, wherein the compounds of the formula I are used in the eye medicament in a concentration (W/V) of 0.001 % to 0.02 %.

**24.** Use according to any one of claims 16-21, wherein the compounds of the formula I are used in the eye medicament in a concentration (W/V) of 0.002 % to 0.015 %.

**25.** A solution for the treatment of contact lenses which is isotonic with human lachrymal fluid, containing at least one compound of the formula I

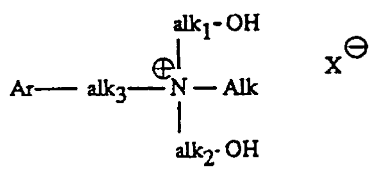

(I)

in which Ar is aryl, with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are $C_1$-$C_7$alkylene and $X^{\ominus}$ is an ophthalmically acceptable anion, and one or more adjuncts which are compatible with contact lenses.

**26.** A solution for the treatment of contact lenses according to claim 25 containing at least one compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride.

**27.** A cleansing solution for contact lenses according to claim 26 containing:
(a) a compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride in a concentration (W/V) of 0.005 to 0.8 %,
(b) an isotonizing agent,
(c) a buffer substance,
(d) a complex-former,
(e) a solubilizer and, if appropriate,
(f) a thickener.

**28.** A conditioner for contact lenses according to claim 26 containing:

(a) a compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride in a concentration (W/V) of 0.002 to 0.8 %,
(b) a moistening agent.
(c) a buffer substance,
(d) an isotonizing agent and, if appropriate,
(e) a complex-former.

**29.** Use of at least one compound of the formula I

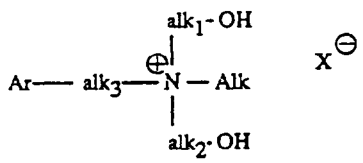

(I)

in which Ar is aryl, with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are $C_1$-$C_7$alkylene and $X^{\ominus}$ is an ophthalmically acceptable anion, for the preservation of contact lenses.

**30.** Use according to claim 29, wherein at least one compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride, is used.

**Claims for the following Contracting State : GR**

**1.** A process for preparing an antimicrobial opthalmic composition which is isotonic with human lachrymal fluid, containing at least one compound of the formula I

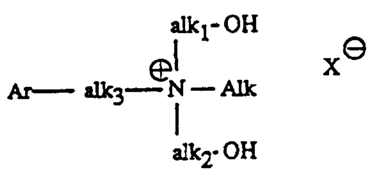

(I)

in which Ar is aryl, with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are alkylene, and $X^{\ominus}$ is an ophthalmically acceptable anion, and one or more adjuncts which are ophthalmically acceptable and compatible with contact lenses, which process comprises mixing the components of the composition with one another.

**2.** A process for preparing an antimicrobial opthalmic composition according to claim 1 containing at least one compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride.

**3.** A process for preparing an eye medicament according to claim 1 containing at least one compound of the formula I

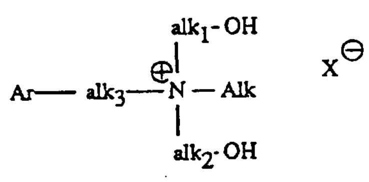

(I)

in which Ar is aryl, with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-

$C_{20}$alkyl, $alk_1$, $alk_2$ and $alk_3$ independently of one another are $C_1$-$C_7$alkylene and $X^{\ominus}$ is an ophthalmically acceptable anion, and one or more ophthalmically acceptable adjuncts, which process comprises mixing the components of the eye medicament with one another.

**4.** A process for preparing an eye medicament according to claim 3 containing at least one compound of the formula I in which Ar is phenyl which is unsubstituted or substituted by $C_1$-$C_7$alkyl, $C_1$-$C_7$-alkoxy, halogen or trifluoromethyl, Alk is $C_1$-$C_{20}$alkyl, $alk_1$ and $alk_2$ independently of one another are $C_2$-$C_4$alkylene, $alk_3$ is $C_1$-$C_4$alkylene and $X^{\ominus}$ is an ophthalmically acceptable anion.

**5.** A process for preparing an eye medicament according to claim 3 containing at least one compound of the formula I in which Ar is phenyl, Alk is $C_1$-$C_{18}$alkyl, $alk_1$ and $alk_2$ are 1,2-ethylene, $alk_3$ is methylene and $X^{\ominus}$ is bromide or chloride.

**6.** A process for preparing an eye medicament according to claim 3 containing at least one compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride.

**7.** A process for preparing an eye medicament according to claim 3 containing N-benzyl-N-(n-dodecyl)-N,N-bis(2-hydroxyethyl)ammonium chloride or N-benzyl-N-(mixed $C_8$-$C_{18}$alkyl)-N,N-bis(2-hydroxyethyl)ammonium chloride.

**8.** A process for preparing an eye medicament according to claim 3 containing N-benzyl-N-(n-dodecyl)-N,N-bis(2-hydroxyethyl)ammonium chloride.

**9.** A process for preparing an eye medicament according to any one of claims 3 to 8, which contains the compounds of the formula I in a concentration (W/V) of 0.0001 % to 0.10 %.

**10.** A process for preparing an eye medicament according to any one of claims 3 to 8, which contains the compounds of the formula I in a concentration (W/V) of 0.001 % to 0.02 %.

**11.** A process for preparing an eye medicament according to any one of claims 3 to 8, which contains the compounds of the formula I in a concentration (W/V) of 0.002 % to 0.015 %.

**12.** A process for preparing an eye medicament according to any one of claims 3-11 containing the pharmaceutical active ingredient diclofenac or an ophthalmically acceptable salt thereof.

**13.** A process for preparing an eye medicament according to any one of claims 3-11 containing the pharmaceutical active ingredient diclofenac sodium.

**14.** A process for preparing an eye medicament according to any one of claims 3-11 containing the pharmaceutical active ingredients diclofenac sodium and gentamicin.

**15.** A process for preparing an eye medicament according to any one of claims 3-11 containing the pharmaceutical active ingredient naphazoline nitrate.

**16.** Use of at least one compound of the formula I

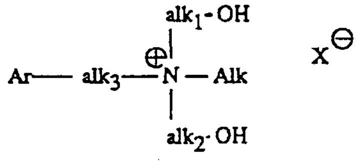

(I)

in which Ar is aryl, with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are $C_1$-$C_7$alkylene, and $X^{\ominus}$ is an ophthalmically acceptable anion, for the preservation of eye medicaments.

**17.** Use according to claim 16, wherein at least one compound of the formula I in which Ar is phenyl which is

unsubstituted or substituted by $C_1$-$C_7$alkyl, $C_1$-$C_7$alkoxy, halogen or trifluoromethyl, Alk is $C_1$-$C_{20}$alkyl, $alk_1$ and $alk_2$ independently of one another are $C_2$-$C_4$alkylene, $alk_3$ is $C_1$-$C_4$alkylene and $X^\ominus$ is an ophthalmically acceptable anion, is used.

**18.** Use according to claim 16, wherein at least one compound of the formula I in which Ar is phenyl, Alk is $C_1$-$C_{18}$alkyl, $alk_1$ and $alk_2$ are 1,2-ethylene, $alk_3$ is methylene and $X^\ominus$ is bromide or chloride, is used.

**19.** Use according to claim 16, wherein at least one compound of the formula I in which Ar is phenyl, Alk is $C_8$-$C_{18}$alkyl, $alk_1$ and $alk_2$ are 1,2-ethylene, $alk_3$ is methylene and $X^\ominus$ is chloride, is used.

**20.** Use according to claim 16, wherein N-benzyl-N-(n-dodecyl)-N,N-bis(2-hydroxyethyl)ammonium chloride or N-benzyl-N-(mixed $C_8$-$C_{18}$alkyl)-N,N-bis(2-hydroxyethyl)-ammonium chloride is used.

**21.** Use according to claim 16, wherein N-benzyl-N-(n-dodecyl)-N,N-bis(2-hydroxyethyl)ammonium chloride is used.

**22.** Use according to any one of claims 16-21, wherein the compounds of the formula I are used in the eye medicament in a concentration (W/V) of 0.0001 % to 0.10 %.

**23.** Use according to any one of claims 16-21, wherein the compounds of the formula I are used in the eye medicament in a concentration (W/V) of 0.001 % to 0.02 %.

**24.** Use according to any one of claims 16-21, wherein the compounds of the formula I are used in the eye medicament in a concentration (W/V) of 0.002 % to 0.015 %.

**25.** A solution for the treatment of contact lenses which is isotonic with human lachrymal fluid, containing at least one compound of the formula I.

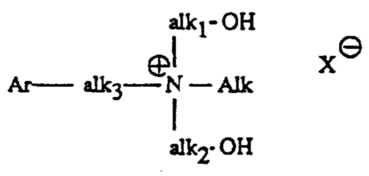

(I)

in which Ar is aryl with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are $C_1$-$C_7$alkylene, and $X^\ominus$ is an ophthalmically acceptable anion, and one or more adjuncts which are compatible with contact lenses.

**26.** A solution for the treatment of contact lenses according to claim 25 containing at least one compound of the formula I in which Ar is phenyl, Alk is $C_8$-$C_{18}$alkyl, $alk_1$ and $alk_2$ are 1,2-ethylene, $alk_3$ is methylene and $X^\ominus$ is chloride.

**27.** A cleansing solution for contact lenses according to claim 26 containing:
(a) a compound of the formula I in which Ar is phenyl, Alk is $C_8$-$C_{18}$alkyl, $alk_1$ and $alk_2$ are 1,2-ethylene, $alk_3$ is methylene and $X^\ominus$ is chloride in a concentration (W/V) of 0.005 to 0.8 %,
(b) an isotonizing agent,
(c) a buffer substance,
(d) a complex-former,
(e) a solubilizer and, if appropriate,
(f) a thickener.

**28.** A conditioner for contact lenses according to claim 26 containing:
(a) a compound of the formula I in which Ar is phenyl, Alk is $C_8$-$C_{18}$alkyl, $alk_1$ and $alk_2$ are 1,2-ethylene, $alk_3$ is methylene and $X\ominus$ is chloride in a concentration (W/V) of 0.002 to 0.8 %,
(b) a moistening agent,
(c) a buffer substance,

(d) an isotonizing agent and, if appropriate,
(e) a complex-former.

**29.** Use of at least one compound of the formula I

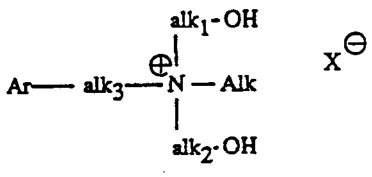

(I)

in which Ar is aryl, with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$ and $alk_2$ and $alk_3$ independently of one another are $C_1$-$C_7$alkylene, and $X^{\ominus}$ is an ophthalmically acceptable anion, for the preservation of contact lenses.

**30.** Use according to claim 29, wherein at least one compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride, is used.

**Claims for the following Contracting State : ES**

**1.** A process for preparing an antimicrobial ophthalmic composition which is isotonic with human lachrymal fluid, containing at least one compound of the formula I

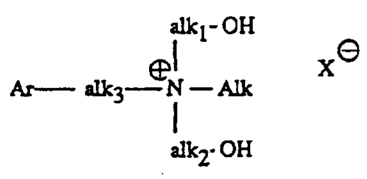

(I)

in which Ar is aryl, with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are $C_1$-$C_7$alkylene, and $X^{\ominus}$ is an ophthalmically acceptable anion, and one or more adjuncts which are ophthalmically acceptable and compatible with contact lenses, which comprises mixing the components of the composition with one another.

**2.** A process for preparing an antimicrobial opthalmic composition according to claim 1 containing at least one compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride.

**3.** A process for preparing an eye medicament according to claim 1 containing at least one compound of the formula I

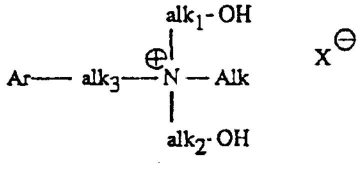

(I)

in which Ar is aryl, with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are $C_1$-$C_7$alkylene and $X^{\ominus}$ is an ophthalmically acceptable anion, and one or more ophthalmically acceptable adjuncts, which comprises mixing the components of the eye medicament with one another.

**4.** A process for preparing an eye medicament according to claim 3 containing at least one compound of the formula I in which Ar is phenyl which is unsubstituted or substituted by $C_1$-$C_7$alkyl, $C_1$-$C_7$-alkoxy, halogen or trifluoromethyl, Alk is $C_1$-$C_{20}$alkyl, $alk_1$ and $alk_2$ independently of one another are $C_2$-$C_4$alkylene, $alk_3$ is $C_1$-$C_4$alkylene and $X^\ominus$ is an ophthalmically acceptable anion.

**5.** A process for preparing an eye medicament according to claim 3 containing at least one compound of the formula I in which Ar is phenyl, Alk is $C_1$-$C_{18}$alkyl, $alk_1$ and $alk_2$ are 1,2-ethylene, $alk_3$ is methylene and $X^\ominus$ is bromide or chloride.

**6.** A process for preparing an eye medicament according to claim 3 containing at least one compound of the formula I in which Ar is phenyl, Alk is $C_8$-$C_{18}$alkyl, $alk_1$ and $alk_2$ are 1,2-ethylene, $alk_3$ is methylene and $X^\ominus$ is chloride.

**7.** A process for preparing an eye medicament according to claim 3 containing N-benzyl-N-(n-dodecyl)-N,N-bis(2-hydroxyethyl)ammonium chloride or N-benzyl-N-(mixed $C_8$-$C_{18}$alkyl)-N,N-bis(2-hydroxyethyl)ammonium chloride.

**8.** A process for preparing an eye medicament according to claim 3 containing N-benzyl-N-(n-dodecyl)-N,N-bis(2-hydroxyethyl)ammonium chloride.

**9.** A process for preparing an eye medicament according to any one of claims 3 to 8, which contains the compounds of the formula I in a concentration (W/V) of 0.0001 % to 0.10 %.

**10.** A process for preparing an eye medicament according to any one of claims 3 to 8, which contains the compounds of the formula I in a concentration (W/V) of 0.001 % to 0.02 %.

**11.** A process for preparing an eye medicament according to any one of claims 3 to 8, which contains the compounds of the formula I in a concentration (W/V) of 0.002 % to 0.015 %.

**12.** A process for preparing an eye medicament according to any one of claims 3-11 containing the pharmaceutical active ingredient diclofenac or an ophthalmically acceptable salt thereof.

**13.** A process for preparing an eye medicament according to any one of claims 3-11 containing the pharmaceutical active ingredient diclofenac sodium.

**14.** A process for preparing an eye medicament according to any one of claims 3-11 containing the pharmaceutical active ingredients diclofenac sodium and gentamicin.

**15.** A process for preparing an eye medicament according to any one of claims 3-11 containing the pharmaceutical active ingredient naphazoline nitrate.

**16.** Use of at least one compound of the formula I

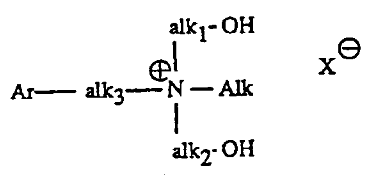

(I)

in which Ar is aryl, with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are $C_1$-$C_7$alkylene and $X^\ominus$ is an ophthalmically acceptable anion, for the preservation of eye medicaments.

**17.** Use according to claim 16, wherein at least one compound of the formula I in which Ar is phenyl which is unsubstituted or substituted by $C_1$-$C_7$alkyl, $C_1$-$C_7$alkoxy, halogen or trifluoromethyl, Alk is $C_1$-$C_{20}$alkyl, $alk_1$ and $alk_2$ independently of one another are $C_2$-$C_4$alkylene, $alk_3$ is $C_1$-$C_4$alkylene and $X^\ominus$ is an ophthalmically acceptable anion, is used.

**18.** Use according to claim 16, wherein at least one compound of the formula I in which Ar is phenyl, Alk is $C_1$-$C_{18}$alkyl, $alk_1$ and $alk_2$ are 1,2-ethylene, $alk_3$ is methylene and $X^{\ominus}$ is bromide or chloride, is used.

**19.** Use according to claim 16, wherein at least one compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride, is used.

**20.** Use according to claim 16, wherein N-benzyl-N-(n-dodecyl)-N,N-bis(2-hydroxyethyl)ammonium chloride or N-benzyl-N-(mixed $C_8$-$C_{18}$alkyl)-N,N-bis(2-hydroxyethyl)ammonium chloride is used.

**21.** Use according to claim 16, wherein N-benzyl-N-(n-dodecyl)-N,N-bis(2-hydroxyethyl)ammonium chloride is used.

**22.** Use according to any one of claims 16-21, wherein the compounds of the formula I are used in the eye medicament in a concentration (W/V) of 0.0001 % to 0.10 %.

**23.** Use according to any one of claims 16-21, wherein the compounds of the formula I are used in the eye medicament in a concentration (W/V) of 0.001 % to 0.02 %.

**24.** Use according to any one of claims 16-21, wherein the compounds of the formula I are used in the eye medicament in a concentration (W/V) of 0.002 % to 0.015 %.

**25.** A process for producing a solution for the treatment of contact lenses which is isotonic with human lachrymal fluid, containing at least one compound of the formula I.

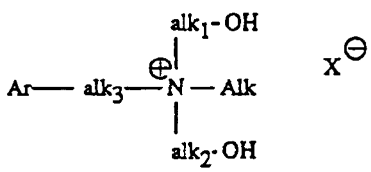

(I)

in which Ar is aryl with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$, $alk_2$ and $alk_3$ independently of one another are $C_1$-$C_7$alkylene, and $X^{\ominus}$ is an ophthalmically acceptable anion, and one or more adjuncts which are compatible with contact lenses, which comprises mixing the components of the solution with one another.

**26.** A process for producing a solution for the treatment of contact lenses according to claim 25 containing at least one compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride.

**27.** A process for producing a cleansing solution for contact lenses according to claim 26 containing:
(a) a compound of the formula I in which Ar is phenyl, Alk is $C_8$-$C_{18}$alkyl, $alk_1$ and $alk_2$ are 1,2-ethylene, $alk_3$ is methylene and $X^{\ominus}$ is chloride in a concentration (W/V) of 0.005 to 0.8 %,
(b) an isotonizing agent,
(c) a buffer substance,
(d) a complex-former,
(e) a solubilizer and, if appropriate,
(f) a thickener.

**28.** A process for producing a conditioner for contact lenses according to claim 26 containing:
(a) a compound of the formula I in which Ar is phenyl, Alk is $C_8$-$C_{18}$alkyl, $alk_1$ and $alk_2$ are 1,2-ethylene, $alk_3$ is methylene and $X\ominus$ is chloride in a concentration (W/V) of 0.002 to 0.8 %,
(b) a moistening agent,
(c) a buffer substance,
(d) an isotonizing agent and, if appropriate,
(e) a complex-former.

**29.** Use of at least one compound of the formula I

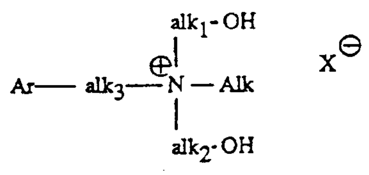

(I)

in which Ar is aryl with aryl being one of the radicals naphthyl and phenyl which, independently of one another, are unsubstituted or substituted by $C_1$-$C_7$alkyl, $C_1$-$C_7$alkoxy, halogen or trifluoromethyl, Alk is $C_1$-$C_{20}$alkyl, $alk_1$ and $alk_2$ and $alk_3$ independently of one another are $C_1$-$C_7$alkylene, and $X^\ominus$ is an ophthalmically acceptable anion, for the preservation of contact lenses.

**30.** Use according to claim 29, wherein at least one compound of the formula I in which Ar is phenyl, Alk is $C_8$-$C_{18}$alkyl, $alk_1$ and $alk_2$ are 1,2-ethylene, $alk_3$ is methylene and $X^\ominus$ is chloride, is used.

## Revendications

### Revendications pour les Etats contractants suivants : AT, BE, CH, DE, DK, FR, GB, IT, LI, LU, NL, SE

**1.** Composition ophtalmique antimicrobienne, isotonique avec le liquide lacrymal humain, contenant au moins un composé de formule I

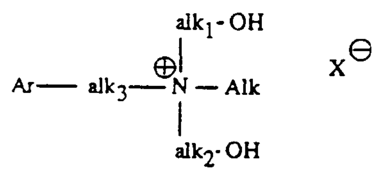

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^\ominus$ représente un anion acceptable du point de vue ophtalmique et un ou plusieurs adjuvants compatibles du point de vue ophtalmique ou compatibles avec les lentilles de contact.

**2.** Composition ophtalmique antimicrobienne selon la revendication 1, contenant au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^\ominus$ représente le chlorure.

**3.** Médicament ophtalmique selon la revendication 1, contenant au moins un composé de formule I

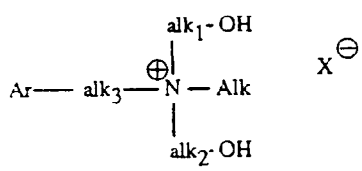

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^\ominus$ représente un anion acceptable du point de vue ophtalmique ou un ou plusieurs adjuvants compatibles du point de vue ophtalmique.

4. Médicament ophtalmique selon la revendication 3, contenant au moins un composé de formule I où Ar représente le phényle, ledit phényle étant non substitué ou substitué par un alkyle en $C_1$-$C_7$, un alcoxy en $C_1$-$C_7$, un halogène ou le trifluorométhyle, Alk représente un alkyle en $C_1$-$C_{20}$, $alk_1$ et $alk_2$ représentent, indépendamment l'un de l'autre, un alkylène en $C_2$-$C_4$, $alk_3$ représente un alkylène en $C_1$-$C_4$ et $X^{\ominus}$ représente un anion acceptable du point de vue ophtalmique.

5. Médicament ophtalmique selon la revendication 3, contenant au moins un composé de formule I où Ar représente le phényle, Alk représente un alkyle en $C_1$-$C_{18}$, $alk_1$ et $alk_2$ représentent le 1,2-éthylène, $alk_3$ représente le méthylène et $X^{\ominus}$ représente le bromure ou le chlorure.

6. Médicament ophtalmique selon la revendication 3, contenant au moins un composé de formule I où Ar représente le phényle,Alk représente un alkyle en $C_8$-$C_{18}$, $alk_1$ et $alk_2$ représentent le 1,2-éthylène, $alk_3$ représente le méthylène et $X^{\ominus}$ représente le chlorure.

7. Médicament ophtalmique selon la revendication 3, contenant du chlorure de N-benzyl-N-(n-dodécyl)-N,N-bis-(2-hydroxyéthyl)-ammonium ou du chlorure de N-benzyl-N-(mélange d'alkyles en $C_8$-$C_{18}$)-N,N-bis-(2-hydroxyéthyl)-ammonium.

8. Médicament ophtalmique selon la revendication 3, contenant du chlorure de N-benzyl-N-(n-dodécyl)-N,N-bis-(2-hydroxyéthyl)-ammonium.

9. Médicament ophtalmique selon l'une des revendications 3 à 8, caractérisé en ce qu'il contient les composés de formule I à une concentration (P/V) allant de 0,0001 % à 0,10 %.

10. Médicament ophtalmique selon l'une des revendications 3 à 8, caractérisé en ce qu'il contient les composés de formule I à une concentration (P/V) allant de 0,001 % à 0,02 %.

11. Médicament ophtalmique selon l'une des revendications 3 à 8, caractérisé en ce qu'il contient les composés de formule I à une concentration (P/V) allant de 0,002 % à 0,015 %.

12. Médicament ophtalmique selon l'une des revendications 3 à 11, contenant la substance active pharmaceutique le Diclofénac ou l'un de ses sels acceptables du point de vue ophtalmique.

13. Médicament ophtalmique selon l'une des revendications 3 à 11, contenant la substance active pharmaceutique le Diclofénac sodique.

14. Médicament ophtalmique selon l'une des revendications 3 à 11, contenant les substances actives pharmaceutiques le Diclofénac sodique et la gentamicine.

15. Médicament ophtalmique selon l'une des revendications 3 à 11, contenant la substance active pharmaceutique le nitrate de naphazoline.

16. Utilisation d'au moins un composé de formule I

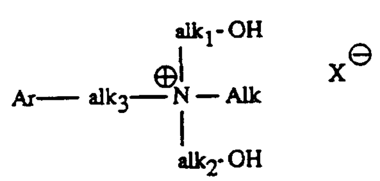

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^{\ominus}$ représente un anion acceptable du point de vue ophtalmique, pour la conservation des médicaments ophtalmiques.

17. Utilisation selon la revendication 16, caractérisée en ce que l'on utilise au moins un composé de formule I où Ar représente le phényle, ledit phényle étant non substitué ou substitué par un alkyle en $C_1$-$C_7$, un alcoxy en $C_1$-$C_7$, un halogène ou le trifluorométhyle, Alk représente un alkyle en $C_1$-$C_{20}$, $alk_1$ et $alk_2$ re-

présentent, indépendamment l'un de l'autre, un alkylène en $C_2$-$C_4$, $alk_3$ représente un alkylène en $C_1$-$C_4$ et $X^{\ominus}$ représente un anion acceptable du point de vue ophtalmique.

**18.** Utilisation selon la revendication 16, caractérisée en ce que l'on utilise au moins un composé de formule I où Ar représente le phényle, Alk représente un alkyle en $C_1$-$C_{18}$, $alk_1$ et $alk_2$ représentent le 1,2-éthylène, $alk_3$ représente le méthylène et $X^{\ominus}$ représente le bromure ou le chlorure.

**19.** Utilisation selon la revendication 16, caractérisée en ce que l'on utilise au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure.

**20.** Utilisation selon la revendication 16, caractérisée en ce que l'on utilise du chlorure de N-benzyl-N-(n-dodécyl)-N,N-bis-(2-hydroxyéthyl)-ammonium ou du chlorure de N-benzyl-N-(mélange d'alkyles en $C_8$-$C_{18}$)-N,N-bis-(2-hydroxyéthyl)-ammonium.

**21.** Utilisation selon la revendication 16, caractérisée en ce que l'on utilise du chlorure de N-benzyl-N-(n-dodécyl)-N,N-bis-(2-hydroxyéthyl)-ammonium.

**22.** Utilisation selon l'une des revendications 16 à 21, caractérisée en ce que l'on utilise les composés de formule I dans le médicament ophtalmique à une concentration (P/V) allant de 0,0001 % à 0,02 %.

**23.** Utilisation selon l'une des revendications 16 à 21, caractérisée en ce que l'on utilise les composés de formule I dans le médicament ophtalmique à une concentration (P/V) allant de 0,001 % à 0,02 %.

**24.** Utilisation selon l'une des revendications 16 à 21, caractérisée en ce que l'on utilise les composés de formule I dans le médicament ophtalmique à une concentration (P/V) allant de 0,002 % à 0,015 %.

**25.** Une solution pour le traitement des lentilles de contact, isotonique avec le liquide lacrymal humain, contenant au moins un composé de formule I

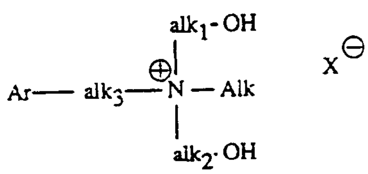

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^{\ominus}$ représente un anion acceptable du point de vue ophtalmique et un ou plusieurs adjuvants compatibles avec les lentilles de contact.

**26.** Une solution pour le traitement des lentilles de contact selon la revendication 25, contenant au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure.

**27.** Une solution de nettoyage pour les lentilles de contact selon la revendication 26 contenant :

(a) un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure, à une concentration (P/V) allant de 0,005 à 0,8 %,
(b) un agent isotonisant,
(c) une substance tampon,
(d) un complexant,
(e) un tiers solvant et éventuellement
(f) un agent épaississant.

**28.** Une solution de conditionnement pour les lentilles de contact selon la revendication 26, contenant

(a) un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure, à une concentration (P/V) allant de 0,002 à 0,8 %,
(b) un agent humectant,
(c) une solution tampon,
(d) un agent isotonisant et éventuellement
(e) un complexant.

**29.** Utilisation d'au moins un composé de formule I

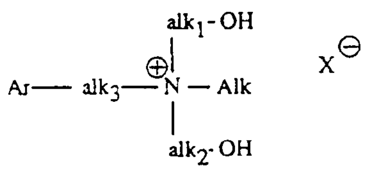

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, alk représente un alkyle en $C_1$-$C_{20}$, $alk_1$, $alk_2$ et $alk_3$ représentent, indépendamment les uns des autres, un alkylène en $C_1$-$C_7$ et $X^{\ominus}$ représente un anion acceptable du point de vue ophtalmique, pour la conservation des lentilles de contact.

**30.** Utilisation selon la revendication 29, caractérisée en ce que l'on utilise au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure.

**Revendications pour l'Etat contractant suivant : GR**

**1.** Procédé pour la préparation d'une composition ophtalmique antimicrobienne, isotonique avec le liquide lacrymal humain, contenant au moins un composé de formule I

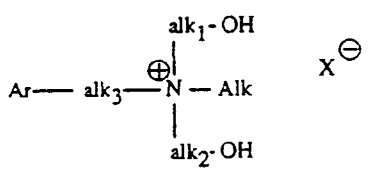

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^{\ominus}$ représente un anion acceptable du point de vue ophtalmique et un ou plusieurs adjuvants compatibles du point de vue ophtalmique ou compatibles avec les lentilles de contact, caractérisé en ce que l'on mélange entre eux les constituants de la composition.

**2.** Procédé pour la préparation d'une composition ophtalmique antimicrobienne selon la revendication 1, contenant au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure.

**3.** Procédé pour la préparation d'un médicament ophtalmique selon la revendication 1, contenant au moins un composé de formule I

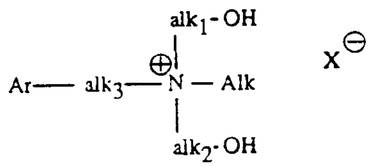

(I)

où Ar représente un aryle, ledit aryle représentant l'un des restes naphtyle ou phényLe, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^{\ominus}$ représente un anion acceptable du point de vue ophtalmique ou un ou plusieurs adjuvants compatibles du point de vue ophtalmique, caractérisé en ce que l'on mélange entre eux les constituants du médicament ophtalmique.

**4.** Procédé pour la préparation d'un médicament ophtalmique selon la revendication 3, contenant au moins un composé de formule I où Ar représente le phényle, ledit phényle étant non substitué ou substitué par un alkyle en $C_1$-$C_7$, un alcoxy en $C_1$-$C_7$, un halogène ou le trifluorométhyle, Alk représente un alkyle en $C_1$-$C_{20}$, $alk_1$ et $alk_2$ représentent, indépendamment l'un de l'autre, un alkylène en $C_2$-$C_4$, $alk_3$ représente un alkylène en $C_1$-$C_4$ et $X^{\ominus}$ représente un anion acceptable du point de vue ophtalmique.

**5.** Procédé pour la préparation d'un médicament ophtalmique selon la revendication 3, contenant au moins un composé de formule I où Ar représente le phényle, Alk représente un alkyle en $C_1$-$C_{18}$, $alk_1$ et $alk_2$ représentent le 1,2-éthylène, $alk_3$ représente le méthylène et $X^{\ominus}$ représente le bromure ou le chlorure.

**6.** Procédé pour la préparation d'un médicament ophtalmique selon la revendication 3, contenant au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure.

**7.** Procédé pour la préparation d'un médicament ophtalmique selon la revendication 3, contenant du chlorure de N-benzyl-N-(n-dodécyl)-N,N-bis-(2-hydroxyéthyl)ammonium ou du chlorure de N-benzyl-N-(mélange d'alkyles en $C_8$-$C_{18}$)-N,N-bis-(2-hydroxyéthyl)-ammonium.

**8.** Procédé pour la préparation d'un médicament ophtalmique selon la revendication 3, contenant du chlorure de N-benzyl-N-(n-dodécyl)-N,N-bis-(2-hydroxyéthyl)ammonium.

**9.** Procédé pour la préparation d'un médicament ophtalmique selon l'une des revendications 3 à 8, caractérisé en ce qu'il contient les composés de formule I à une concentration (P/V) allant de 0,0001 % à 0,10 %.

**10.** Procédé pour la préparation d'un médicament ophtalmique selon l'une des revendications 3 à 8, caractérisé en ce qu'il contient les composés de formule I à une concentration (P/V) allant de 0,001 % à 0,02 %.

**11.** Procédé pour la préparation d'un médicament ophtalmique selon l'une des revendications 3 à 8, caractérisé en ce qu'il contient les composés de formule I à une concentration (P/V) allant de 0,002 % à 0,015 %.

**12.** Procédé pour la préparation d'un médicament ophtalmique selon l'une des revendications 3 à 11, contenant la substance active pharmaceutique le Diclofénac ou l'un de ses sels acceptables du point de vue ophtalmique.

**13.** Procédé pour la préparation d'un médicament ophtalmique selon l'une des revendications 3 à 11, contenant la substance active pharmaceutique le Diclofénac sodique.

**14.** Procédé pour la préparation d'un médicament ophtalmique selon l'une des revendications 3 à 11, contenant les substances actives pharmaceutiques le Diclofénac sodique et la gentamicine.

**15.** Procédé pour la préparation d'un médicament ophtalmique selon l'une des revendications 3 à 11, contenant la substance active pharmaceutique le nitrate de naphazoline.

**16.** Utilisation d'au moins un composé de formule I

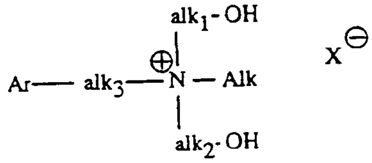

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^{\ominus}$ représente un anion acceptable du point de vue ophtalmique, pour la conservation des médicaments ophtalmiques.

**17.** Utilisation selon la revendication 16, caractérisée en ce que l'on utilise au moins un composé de formule I où Ar représente le phényle, ledit phényle étant non substitué ou substitué par un alkyle en $C_1$-$C_7$, un alcoxy en $C_1$-$C_7$, un halogène ou le trifluorométhyle, Alk représente un alkyle en $C_1$-$C_{20}$, $alk_1$ et $alk_2$ représentent, indépendamment l'un de l'autre, un alkylène en $C_2$-$C_4$, $alk_3$ représente un alkylène en $C_1$-$C_4$ et $X^{\ominus}$ représente un anion acceptable du point de vue ophtalmique.

**18.** Utilisation selon la revendication 16, caractérisée en ce que l'on utilise au moins un composé de formule I où Ar représente le phényle, Alk représente un alkyle en $C_1$-$C_{18}$, $alk_1$ et $alk_2$ représentent le 1,2-éthylène, $alk_3$ représente le méthylène et $X^{\ominus}$ représente le bromure ou le chlorure.

**19.** Utilisation selon la revendication 16, caractérisée en ce que l'on utilise au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure.

**20.** Utilisation selon la revendication 16, caractérisée en ce que l'on utilise du chlorure de N-benzyl-N-(n-dodécyl)-N,N-bis-(2-hydroxyéthyl)-ammonium ou du chlorure de N-benzyl-N-(mélange d'alkyles en $C_8$-$C_{18}$)-N,N-bis-(2-hydroxyéthyl)-ammonium.

**21.** Utilisation selon la revendication 16, caractérisée en ce que l'on utilise du chlorure de N-benzyl-N-(n-dodécyl)-N,N-bis-(2-hydroxyéthyl)-ammonium.

**22.** Utilisation selon l'une des revendications 16 à 21, caractérisée en ce que l'on utilise les composés de formule I dans le médicament ophtalmique à une concentration (P/V) allant de 0,0001 % à 0,02 %.

**23.** Utilisation selon l'une des revendications 16 à 21, caractérisée en ce que l'on utilise les composés de formule I dans le médicament ophtalmique à une concentration (P/V) allant de 0,001 % à 0,02 %.

**24.** Utilisation selon l'une des revendications 16 à 21, caractérisée en ce que l'on utilise les composés de formule I dans le médicament ophtalmique à une concentration (P/V) allant de 0,002 % à 0,015 %.

**25.** Une solution pour le traitement des lentilles de contact, isotonique avec le liquide lacrymal humain, contenant au moins un composé de formule I

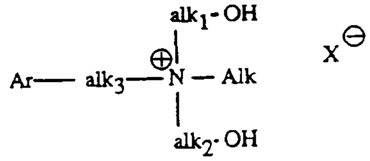

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$

représentent, indépendamment les uns des autres, un alkylène en $C_1$-$C_7$ et $X^\ominus$ représente un anion acceptable du point de vue ophtalmique et un ou plusieurs adjuvants compatibles avec les lentilles de contact.

**26.** Une solution pour le traitement des lentilles de contact selon la revendication 25, contenant au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^\ominus$ représente le chlorure.

**27.** Une solution de nettoyage pour les lentilles de contact selon la revendication 26 contenant :
(a) un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^\ominus$ représente le chlorure, à une concentration (P/V) allant de 0,005 à 0,8 %,
(b) un agent isotonisant,
(c) une substance tampon,
(d) un complexant,
(e) un tiers solvant et éventuellement
(f) un agent épaississant.

**28.** Une solution de conditionnement pour les lentilles de contact selon la revendication 26, contenant
(a) un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^\ominus$ représente le chlorure, à une concentration (P/V) allant de 0,002 à 0,8 %,
(b) un agent humectant,
(c) une solution tampon,
(d) un agent isotonisant et éventuellement
(e) un complexant.

**29.** Utilisation d'au moins un composé de formule I

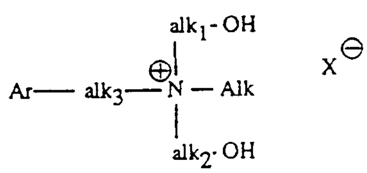

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^\ominus$ représente un anion acceptable du point de vue ophtalmique, pour la conservation des lentilles de contact.

**30.** Utilisation selon la revendication 29, caractérisée en ce que l'on utilise au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^\ominus$ représente le chlorure.

**Revendications pour l'Etat contractant suivant : ES**

**1.** Procédé pour la préparation d'une composition ophtalmique antimicrobienne, isotonique avec le liquide lacrymal humain, contenant au moins un composé de formule I

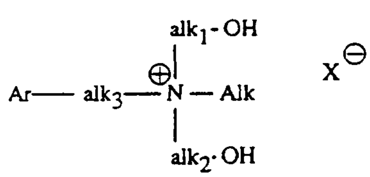

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^\ominus$ représente un anion acceptable du point de vue ophtalmique et un ou plusieurs adjuvants compatibles du point de vue ophtalmique ou compatibles avec les lentilles de contact, caractérisé en ce que l'on mélange entre eux les constituants de la composition.

2. Procédé pour la préparation d'une composition ophtalmique antimicrobienne selon la revendication 1, contenant au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^\ominus$ représente le chlorure.

3. Procédé pour la préparation d'un.médicament ophtalmique selon la revendication 1, contenant au moins un composé de formule I

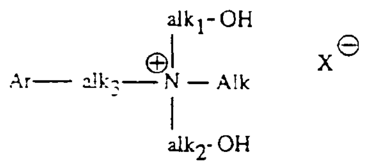

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^\ominus$ représente un anion acceptable du point de vue ophtalmique ou un ou plusieurs adjuvants compatibles du point de vue ophtalmique, caractérisé en ce que l'on mélange entre eux les constituants du médicament ophtalmique.

4. Procédé de préparation d'un médicament ophtalmique selon la revendication 3, contenant au moins un composé de formule I où Ar représente le phényle, ledit phényle étant non substitué ou substitué par un alkyle en $C_1$-$C_7$, un alcoxy en $C_1$-$C_7$, un halogène ou le trifluorométhyle, Alk représente un alkyle en $C_1$-$C_{20}$, $alk_1$ et $alk_2$ représentent, indépendamment l'un de l'autre, un alkylène en $C_2$-$C_4$, $alk_3$ représente un alkylène en $C_1$-$C_4$ et $X^\ominus$ représente un anion acceptable du point de vue ophtalmique.

5. Procédé pour la préparation d'un médicament ophtalmique selon la revendication 3, contenant au moins un composé de formule I où Ar représente le phényle, Alk représente un alkyle en $C_1$-$C_{18}$, $alk_1$ et $alk_2$ représentent le 1,2-éthylène, $alk_3$ représente le méthylène et $X^\ominus$ représente le bromure ou le chlorure.

6. Procédé pour la préparation d'un médicament ophtalmique selon la revendication 3, contenant au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^\ominus$ représente le chlorure.

7. Procédé pour la préparation d'un médicament ophtalmique selon la revendication 3, contenant du chlorure de N-benzyl-N-(n-dodécyl)-N,N-bis-(2-hydroxyéthyl)ammonium ou du chlorure de N-benzyl-N-(mélange d'alkyles en $C_8$-$C_{18}$)-N,N-bis-(2-hydroxyéthyl)-ammonium.

8. Procédé pour la préparation d'un médicament ophtalmique selon la revendication 3, contenant du chlorure de N-benzyl-N-(n-dodécyl)-N,N-bis-(2-hydroxyéthyl)ammonium.

9. Procédé pour la préparation d'un médicament ophtalmique selon l'une des revendications 3 à 8, caractérisé en ce qu'il contient les composés de formule I à une concentration (P/V) allant de 0,0001 % à 0,10 %.

10. Procédé pour la préparation d'un médicament ophtalmique selon l'une des revendications 3 à 8, caractérisé en ce qu'il contient les composés de formule I à une concentration (P/V) allant de 0,001 % à 0,02 %.

11. Procédé pour la préparation d'un médicament ophtalmique selon l'une des revendications 3 à 8, caractérisé en ce qu'il contient les composés de formule I à une concentration (P/V) allant de 0,002 % à 0,015 %.

**12.** Procédé pour la préparation d'un médicament ophtalmique selon l'une des revendications 3 à 11, contenant la substance active pharmaceutique le Diclofénac ou l'un de ses sels acceptables du point de vue ophtalmique.

**13.** Procédé pour la préparation d'un médicament ophtalmique selon l'une des revendications 3 à 11, contenant la substance active pharmaceutique le Diclofénac sodique.

**14.** Procédé pour la préparation d'un médicament ophtalmique selon l'une des revendications 3 à 11, contenant les substances actives pharmaceutiques le Diclofénac sodique et la gentamicine.

**15.** Procédé pour la préparation d'un médicament ophtalmique selon l'une des revendications 3 à 11, contenant la substance active pharmaceutique le nitrate de naphazoline.

**16.** Utilisation d'au moins un composé de formule I

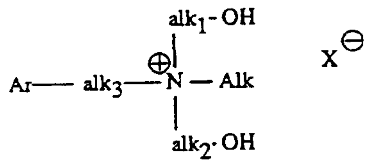

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^{\ominus}$ représente un anion acceptable du point de vue ophtalmique, pour la conservation des médicaments ophtalmiques.

**17.** Utilisation selon la revendication 16, caractérisée en ce que l'on utilise au moins un composé de formule I où Ar représente le phényle, ledit phényle étant non substitué ou substitué par un alkyle en $C_1$-$C_7$, un alcoxy en $C_1$-$C_7$, un halogène ou le trifluorométhyle, Alk représente un alkyle en $C_1$-$C_{20}$, $alk_1$ et $alk_2$ représentent, indépendamment l'un de l'autre, un alkylène en $C_2$-$C_4$, $alk_3$ représente un alkylène en $C_1$-$C_4$ et $X^{\ominus}$ représente un anion acceptable du point de vue ophtalmique.

**18.** Utilisation selon la revendication 16, caractérisée en ce que l'on utilise au moins un composé de formule I où Ar représente le phényle, Alk représente un alkyle en $C_1$-$C_{18}$, $alk_1$ et $alk_2$ représentent le 1,2-éthylène, $alk_3$ représente le méthylène et $X^{\ominus}$ représente le bromure ou le chlorure.

**19.** Utilisation selon la revendication 16, caractérisée en ce que l'on utilise au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure.

**20.** Utilisation selon la revendication 16, caractérisée en ce que l'on utilise du chlorure de N-benzyl-N-(n-dodécyl)-N,N-bis-(2-hydroxyéthyl)-ammonium ou du chlorure de N-benzyl-N-(mélange d'alkyles en $C_8$-$C_{18}$)-N,N-bis-(2-hydroxyéthyl)-ammonium.

**21.** Utilisation selon la revendication 16, caractérisée en ce que l'on utilise du chlorure de N-benzyl-N-(n-dodécyl)-N,N-bis-(2-hydroxyéthyl)-ammonium.

**22.** Utilisation selon l'une des revendications 16 à 21, caractérisée en ce que l'on utilise les composés de formule I dans le médicament ophtalmique à une concentration (P/V) allant de 0,0001 % à 0,02 %.

**23.** Utilisation selon l'une des revendications 16 à 21, caractérisée en ce que l'on utilise les composés de formule I dans le médicament ophtalmique à une concentration (P/V) allant de 0,001 % à 0,02 %.

**24.** Utilisation selon l'une des revendications 16 à 21, caractérisée en ce que l'on utilise les composés de formule I dans le médicament ophtalmique à une concentration (P/V) allant de 0,002 % à 0,015 %.

**25.** Procédé pour la préparation d'une solution pour le traitement des lentilles de contact, isotonique avec le liquide lacrymal humain, contenant au moins un composé de formule I

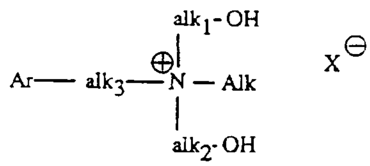

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^{\ominus}$ représente un anion acceptable du point de vue ophtalmique et un ou plusieurs adjuvants compatibles avec les lentilles de contact, caractérisé en ce que l'on mélange entre eux les constituants de la solution.

**26.** Procédé pour la préparation d'une solution pour le traitement des lentilles de contact selon la revendication 25, contenant au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure.

**27.** Procédé pour la préparation d'une solution de nettoyage pour les lentilles de contact selon la revendication 26 contenant :

(a) un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure, à une concentration (P/V) allant de 0,005 à 0,8 %,
(b) un agent isotonisant,
(c) une substance tampon,
(d) un complexant,
(e) un tiers solvant et éventuellement
(f) un agent épaississant.

**28.** Procédé pour la préparation d'une solution de conditionnement pour les lentilles de contact selon la revendication 26, contenant

(a) un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure, à une concentration (P/V) allant de 0,002 à 0,8 %,
(b) un agent humectant,
(c) une solution tampon,
(d) un agent isotonisant et éventuellement
(e) un complexant.

**29.** Utilisation d'au moins un composé de formule I

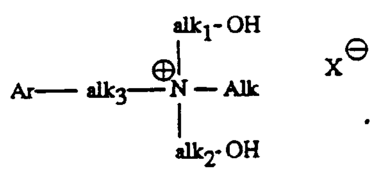

(I)

où Ar représente un aryle, ledit aryle représentant l'un des restes naphtyle ou phényle, lesdits restes naphtyle ou phényle étant, indépendamment l'un de l'autre, non substitués ou substitués par un alkyle en $C_1$-$C_7$, un alcoxy en $C_1$-$C_7$, un halogène ou le trifluorométhyle, Alk représente un alkyle en $C_1$-$C_{20}$, $alk_1$, $alk_2$ et $alk_3$ représentent, indépendamment les uns des autres, un alkylène en $C_1$-$C_7$ et $X^{\ominus}$ représente un anion acceptable du point de vue ophtalmique, pour la conservation des lentilles de contact.

**30.** Utilisation selon la revendication 29, caractérisée en ce que l'on utilise au moins un composé de formule I où Ar représente le phényle, Alk représente un alkyle en $C_8$-$C_{18}$, $alk_1$ et $alk_2$ représentent le 1,2-éthylène, $alk_3$ représente le méthylène et $X^{\ominus}$ représente le chlorure.